(19) Europäisches Patentamt / European Patent Office / Office européen des brevets

(11) **EP 1 930 436 B1**

(12) **EUROPEAN PATENT SPECIFICATION**

(45) Date of publication and mention
of the grant of the patent:
**27.04.2011 Bulletin 2011/17**

(21) Application number: **06810956.0**

(22) Date of filing: **29.09.2006**

(51) Int Cl.:
*C12N 15/09* (2006.01)    *A61K 9/127* (2006.01)
*A61K 47/42* (2006.01)    *A61K 47/44* (2006.01)
*A61K 48/00* (2006.01)

(86) International application number:
**PCT/JP2006/319601**

(87) International publication number:
**WO 2007/037444 (05.04.2007 Gazette 2007/14)**

(54) **VECTOR FOR DELIVERING TARGET SUBSTANCE INTO NUCLEUS OR CELL**

VEKTOR ZUR ZUFÜHRUNG EINER ZIELSUBSTANZ IN DEN ZELLKERN ODER DIE ZELLE

VECTEUR DESTINÉ À LA DÉLIVRANCE D'UNE SUBSTANCE CIBLE DANS UN NOYAU OU UNE CELLULE

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.09.2005 JP 2005289411**

(43) Date of publication of application:
**11.06.2008 Bulletin 2008/24**

(73) Proprietors:
• **National University Corporation Hokkaido University**
**Sapporo-shi, Hokkaido 060-0808 (JP)**
• **SHIONOGI & CO., LTD.**
**Osaka 541-0045 (JP)**

(72) Inventors:
• **AKITA, Hidetaka**
**National University Corp. Hokkaido University**
**Sapporo-shi**
**Hokkaido 060-0812 (JP)**
• **KUDO, Asako**
**National University Corp. Hokkaido University**
**Sapporo-shi**
**Hokkaido 060-0812 (JP)**
• **HARASHIMA, Hideyoshi**
**National University Corp. Hokkaido University**
**Sapporo-shi**
**Hokkaido 060-0812 (JP)**

(74) Representative: **Hiebl, Inge Elisabeth et al**
**Kraus & Weisert**
**Patent- und Rechtsanwälte**
**Thomas-Wimmer-Ring 15**
**80539 München (DE)**

(56) References cited:
**EP-A- 1 847 315     JP-A- 2002 538 096**

• **PATIL SIDDHESH D ET AL: "Anionic liposomal delivery system for DNA transfection." THE AAPS JOURNAL 2004, vol. 6, no. 4, 2004, page e29, XP002495787 ISSN: 1550-7416**
• **MORIGUCHI ET AL: "A multifunctional envelope-type nano device for novel gene delivery of siRNA plasmids" INTERNATIONAL JOURNAL OF PHARMACEUTICS, ELSEVIER BV, NL, vol. 301, no. 1-2, 14 September 2005 (2005-09-14), pages 277-285, XP005041888 ISSN: 0378-5173**
• **KOGURE K. ET AL.: 'Development of a non-viral multifunctional envelope-type nano device by a novel liquid film hydration method' J. CONTROL. RELEASE vol. 98, 2004, pages 317 - 323, XP004521720**

SEQUENCE LISTING

<110> National University Corporation Hokkaido University, et al.

<120> vector for delivering target substance into nucleus or cell

<130> 18594EP

<140> EP 06 810 956.0
<141> 2006-09-29

<150> JP 2005-0289411
<151> 2005-09-30

<160> 3

<170> PatentIn version 3.3

<210> 1
<211> 13
<212> PRT
<213> HIV-1

<220>
<221> PEPTIDE
<222> (1)..(13)
<223> HIV-1-derived Tat (48-60)

<400> 1

EP 1 930 436 B1

Gly Arg Lys Lys Arg Arg Gln Arg Arg Arg Pro Pro Gln
1 5 10

<210> 2
<211> 17
<212> PRT
<213> HIV-1

<220>
<221> PEPTIDE
<222> (1)..(17)
<223> HIV-1-derived Rev (34-50)

<400> 2

Thr Arg Gln Ala Arg Arg Asn Arg Arg Arg Arg Trp Arg Glu Arg Gln
1 5 10 15

Arg

<210> 3
<211> 8
<212> PRT
<213> Artificial

<220>
<223> strongly basic peptide

<220>
<221> MOD_RES
<222> (1)..(1)
<223> N-terminal acylation with CH3(CH2)16C(O)-

<400> 3

Arg Arg Arg Arg Arg Arg Arg Arg
1 5

## Description

TECHNICAL FIELD

The present invention relates to a vector for delivering a target substance into a nucleus or a cell.

BACKGROUND ART

Vectors and carriers for reliably delivering drugs, nucleic acids, peptides, proteins, sugars, and the like to target sites are being actively developed. For example, in the field of gene therapy, viral vectors such as retroviral vectors, adenoviral vectors, and adeno-associated viral vectors have been developed as vectors for introducing desired genes into target cells. However, such viral vectors have various problems such as difficulty in mass production, antigenicity, and toxicity. For this reason, liposomal vectors and peptide carriers having fewer problems than viral vectors are receiving attention. In addition, liposomal vectors have an advantage that their directionality toward target sites can be improved by introducing functional molecules such as antibodies, proteins, and sugar chains to their surfaces.

Nuclear membrane permeability is a very significant bottleneck in development of effective vectors for use in gene therapy. Transport of substances between the cytoplasm and the nucleus occurs through nuclear pore complexes present in the nuclear membrane, and therefore low-molecular weight proteins and ions can permeate the nuclear membrane by free diffusion through nuclear pore complexes. However, polymers cannot freely permeate the nuclear membrane because of their size. For this reason, it is difficult to efficiently deliver genes into nuclei simply by directly introducing the genes into cells.

Meanwhile, in recent years, it has been reported that by modifying the surface of a liposome entrapping condensed DNA with stearylated octaarginine, the cellular entry efficiency of condensed DNA is increased 1000-fold and the cellular entry efficiency of the liposome entrapping condensed DNA is increased 100-fold (see Non-patent Document 1). Further, it has been also reported that a liposome having a surface modified with stearylated octaarginine can enter a cell with its original form being kept intact (see Non-patent Documents 1 and 2).

Non-patent Document 1: Kogure et al., "Journal of Controlled Release", vol. 98, pp. 317-323, 2004
Non-patent Document 2: Khalil Ikramy et al., "YAKUGAKU ZASSHI", vol. 124, suppl. 4, pp. 113-116, 2004

Kogure K. et al.: "Development of a non-viral multifunctional envelope-type nano device by a novel liquid film hydration method", J. Control. Release, vol. 98, 2004, pp. 317-323 discloses the development of a multifunctional envelope-type nano device for use in a non-viral gene delivery system using a novel lipid film hydration method.

Patil S. D. et al.: "Anionic liposomal delivery system for DNA transfection", The AAPS Journal, 2004, vol. 6, no. 4, 2004, page e29 discloses the use of novel anionic lipoplexes composed of physiological components for plasmid DNA delivery into mammalian cells in vitro. Anionic lipoplexes were formed by complexation between anionic liposomes and plasmid DNA molecules encoding green fluorescence protein using $Ca^{2+}$ ions.

Moriguchi R. et al.: "A multifunctional envelope-type nano device for novel gene delivery of siRNA plasmids", International Journal of Pharmaceutics, vol. 301, no. 1-2, 2005, pp. 277-285 discloses a multifunctional envelope-type nano device for use in the delivery of siRNA expression plasmids.

DISCLOSURE OF THE INVENTION

PROBLEMS TO BE SOLVED BY THE INVENTION

An object of the present invention is to provide a vector for delivering a target substance into a nucleus or a cell.

MEANS FOR SOLVING THE PROBLEM

In order to achieve the above object, the present invention is directed to a vector for delivering a target substance into a nucleus, comprising a lipid membrane structure having a first lipid membrane containing an anionic lipid and a second lipid membrane containing an anionic lipid being provided outside the first lipid membrane, wherein the anionic lipid of the first membrane is selected from a group consisting of cholesteryl hemisuccinate, phosphatidic acid and cardiolipin and the anionic lipid of the second membrane is selected from a group consisting of phosphatidic acid, cardiolipin, dialkyl phosphate and diacyl phosphate, and the first lipid membrane and the second lipid membrane contain a membrane permeable peptide.

Therefore, after the vector is introduced into a cell as an endosomal fraction, a macropinosomal fraction, or the like via a pathway such as endocytosis, macropinocytosis, or the like, the second lipid membrane of the vector is bound to and fused with an endosomal membrane, a macropinosomal membrane, or the like so that a target substance contained in the vector escapes from an endosome, a macropinosome, or the like and is efficiently transferred into the cell.

The anionic lipid contained in the first lipid membrane of the vector is cholesteryl hemisuccinate, phosphatidic acid, or cardiolipin. This makes it possible to improve the ability of the first lipid membrane to bind to and fuse with a nuclear membrane.

The amount of the anionic lipid contained in the first lipid membrane of the vector is preferably 20 to 80% (mole ratio) of the total amount of lipids contained in the first lipid membrane. This makes it possible to improve the ability of the first lipid membrane to bind to and fuse with a nuclear membrane.

The anionic lipid contained in the first lipid membrane of the vector is preferably phosphatidic acid or cardiolipin. In this case, the amount of the anionic lipid contained in the first lipid membrane is preferably 40 to 60% (mole ratio) of the total amount of lipids contained in the first lipid membrane. This makes it possible to improve the ability of the first lipid membrane to bind to and fuse with a nuclear membrane.

The first lipid membrane of the vector preferably contains dioleoylphosphatidyl ethanolamine. This makes it possible to improve the ability of the first lipid membrane to bind to and fuse with a nuclear membrane.

The amount of dioleoylphosphatidyl ethanolamine contained in the first lipid membrane of the vector is preferably 20 to 80% (mole ratio) of the total amount of lipids contained in the first lipid membrane. This makes it possible to improve the ability of the first lipid membrane to bind to and fuse with a nuclear membrane.

The first lipid membrane of the vector has a membrane-permeable peptide. This makes it possible to improve the ability of the first lipid membrane to bind to and fuse with a nuclear membrane.

The membrane-permeable peptide of the vector is a peptide having, for example, a protein transduction domain. The protein transduction domain is preferably polyarginine, and the polyarginine is preferably composed of 4 to 20 consecutive arginine residues. By allowing the first lipid membrane to have such polyarginine as a protein transduction domain, it is possible to improve the ability of the first lipid membrane to bind to and fuse with a nuclear membrane. Further, in a case where the first lipid membrane provides the surface of the vector and has polyarginine as a protein transduction domain, it is possible to allow the vector to be transferred into a cell mainly via macropinocytosis by adjusting the amount of polyarginine present on the surface of the vector. In macropinocytosis, an extracellular substance is introduced into a cell as a fraction called "macropinosome" . Such a macropinosome is different from an endosome in that it is not fused with a lysosome, and therefore it is possible to avoid decomposition of a substance entrapped in a macropinosome by a lysosome. For this reason, when the vector is transferred into a cell mainly via macropinocytosis, a target substance contained in the vector is efficiently delivered into a cell.

The membrane-permeable peptide of the vector is preferably present on the surface of the first lipid membrane. By allowing the membrane-permeable peptide to be present on the surface of the first lipid membrane, it is possible to improve the ability of the first lipid membrane to bind to and fuse with a nuclear membrane. Further, in a case where the first lipid membrane provides the surface of the vector and polyarginine is present as a protein transduction domain on the surface of the first lipid membrane, the vector is transferred into a cell mainly via macropinocytosis so that a target substance contained in the vector is efficiently delivered into the cell.

The anionic lipid contained in the second lipid membrane of the vector is phosphatidic acid, cardiolipin, dialkyl phosphate, or diacyl phosphate. This makes it possible to improve the ability of the second lipid membrane to bind to and fuse with an endosomal membrane or a macropinosomal membrane.

The amount of the anionic lipid contained in the second lipid membrane of the vector is preferably 10 to 90% (mole ratio) of the total amount of lipids contained in the second lipid membrane. This makes it possible to improve the ability of the second lipid membrane to bind to and fuse with an endosomal membrane or a macropinosomal membrane.

The second lipid membrane of the vector preferably contains dioleoylphosphatidyl ethanolamine. This makes it possible to improve the ability of the second lipid membrane to bind to and fuse with an endosomal membrane or a macropinosomal membrane.

The amount of dioleoylphosphatidyl ethanolamine contained in the second lipid membrane of the vector is preferably 10 to 90% (mole ratio) of the total amount of lipids contained in the second lipid membrane. This makes it possible to improve the ability of the second lipid membrane to bind to and fuse with an endosomal membrane or a macropinosomal membrane.

The second lipid membrane of the vector has a membrane-permeable peptide. This makes it possible to improve the ability of the second lipid membrane to bind to and fuse with an endosomal membrane or a macropinosomal membrane.

The membrane-permeable peptide of the vector is a peptide having, for example, a protein transduction domain. The protein transduction domain is preferably polyarginine, and the polyarginine is preferably composed of 4 to 20 consecutive arginine residues. By allowing the second lipid membrane to have polyarginine as a protein transduction domain, it is possible to improve the ability of the second lipid membrane to bind to and fuse with an endosomal membrane or a macropinosomal membrane. Further, in a case where the second lipid membrane provides the surface of the vector and has polyarginine as a protein transduction domain, it is possible to allow the vector to be transferred into a cell mainly via macropinocytosis by adjusting the amount of polyarginine present on the surface of the vector. In macropinocytosis, an extracellular substance is introduced into a cell as a fraction called "macropinosome". Such a macropinosome is different from an endosome in that it is not fused with a lysosome, and therefore it is possible to avoid decomposition of a substance entrapped in a macropinosome by a lysosome. For this reason, when the vector is transferred into a cell mainly via macropinocytosis, a target substance contained in the vector is efficiently delivered into a cell.

The membrane-permeable peptide of the vector is preferably present on the surface of the second lipid membrane. By allowing the membrane-permeable peptide to be present on the surface of the second lipid membrane, it is possible to improve the ability of the second lipid membrane to bind to and fuse with an endosomal membrane or a macropinosomal membrane. Further, in a case where the second lipid membrane constitutes the surface of the vector and polyarginine is present as a protein transduction domain on the surface of the second lipid membrane, the vector is transferred into a cell mainly via macropinocytosis so that a target substance contained in the vector is efficiently delivered into the cell.

The lipid membrane structure of the vector is preferably a liposome. In a case where the lipid membrane structure is a liposome, a target substance is entrapped in the lipid membrane structure, thereby making it possible to efficiently deliver the target substance into a cell or a nucleus.

EFFECT OF THE INVENTION

According to the present invention, it is possible to provide a vector for delivering a target substance into a nucleus or a cell.

BRIEF DESCRIPTION OF THE DRAWINGS

Fig. 1 is a diagram showing the binding activity (%) of liposomes to nuclear membranes;
Fig. 2 is a diagram showing the binding activity (%) of liposomes to nuclear membranes;
Fig. 3 is a diagram showing the fusion activity (TF(%)) of liposomes with nuclear membranes;
Fig. 4 is a diagram showing the binding activity (%) of liposomes to nuclear membranes;
Fig. 5 is a diagram showing the fusion activity (TF(%)) of liposomes with nuclear membranes;
Fig. 6 is a diagram showing the correlation between the ability of liposomes to bind to nuclear membranes and their ability to fuse with nuclear membranes;
Fig. 7 is a diagram showing the results of observation by a confocal laser microscope;
Fig. 8 is a diagram showing the fusion activity (TF(%)) of liposomes with nuclear membranes;
Fig. 9 is a diagram showing the fusion activity (TF(%)) of liposomes with nuclear membranes;
Fig. 10 is a diagram showing the results of observation by a confocal laser microscope;
Fig. 11 is a diagram showing the endosomal escape efficiency of liposomes;
Fig. 12 is a diagram showing the expression activity of a gene delivered by liposomes;
Fig. 13 is a diagram showing the results of observation by a confocal laser microscope;
Fig. 14 is a diagram showing the expression activity of a gene delivered by liposomes;
Fig. 15 is a diagram showing the expression activity of a gene delivered by liposomes;
Fig. 16 is a diagram showing the expression activity of a gene delivered by liposomes; and
Fig. 17 is a fragmentary sectional view schematically showing embodiments of a liposome lipid membrane structure according to the present invention.

DESCRIPTION OF THE REFERENCE NUMERALS

| 1a, 1b, 1c ,1d | liposome, |
| 2a, 21b, 22b, 2c, 21d, 23d | lipid membrane |
| 3 | target substance |

BEST MODE FOR CARRYING OUT THE INVENTION

Hereinbelow, a vector according to the present invention will be described in detail.

The lipid membrane structure may be any one of a liposome, an O/W-type emulsion, a W/O/W-type emulsion, a spherical micelle, a wormlike micelle, a layered structure having no regular shape, and the like, but is preferably a liposome. In a case where the lipid membrane structure is a liposome, a target substance is entrapped in the lipid membrane structure, thereby making it possible to efficiently deliver the target substance into a cell.

In a case where the lipid membrane structure is a liposome, the liposome may be either a multilamellar vesicle (MLV) or a unilamellar liposome such as SUV (small unilamellar vesicle), LUV (large unilamellar vesicle), or GUV (giant unilamellar vesicle), but is preferably a multilamellar vesicle. By allowing the liposome to have two or more first lipid membranes, it is possible for the liposome to permeate two nuclear membranes (outer membrane and inner membrane). Further, by allowing the liposome to have an anionic lipid-containing second lipid membrane provided outside the first lipid membrane, it is possible for the liposome to permeate an endosomal membrane or a macropinosomal membrane.

The number of first lipid membranes of the lipid membrane structure is not particularly limited, but is usually 1 to 5, preferably 1 to 3, more preferably 2. The number of second lipid membranes of the lipid membrane structure is not particularly limited, but is usually 1 to 5, preferably 1 to 3, more preferably 1. The lipid membrane structure may also have a lipid membrane other than the first and second lipid membranes.

The size of the lipid membrane structure is not particularly limited. In a case where the lipid membrane structure is a liposome or an emulsion, the particle size thereof is usually in the range of 50 nm to 5 $\mu$m. In a case where the lipid membrane structure is a spherical micelle, the particle size thereof is usually in the range of 5 to 100 nm. In a case where the lipid membrane structure is a wormlike micelle or a layered structure having no regular shape, the thickness of one layer thereof is usually in the range of 5 to 10 nm, and two or more such layers are preferably stacked.

Examples of components of the lipid membranes (including the first lipid membrane, the second lipid membrane, and a lipid membrane other than the first and second lipid membranes) of the lipid membrane structure include lipids, membrane stabilizers, antioxidants, charged substances, and membrane proteins.

Lipid is an essential component of the lipid membrane. The amount of lipid contained in the lipid membrane is usually 70% (mole ratio) or more, preferably 75% (mole ratio) or more, more preferably 80% (mole ratio) or more of the total amount of substances constituting the lipid membrane. It is to be noted that the upper limit value of the amount of lipid contained in the lipid membrane is 100% of the total amount of substances constituting the lipid membrane.

Examples of lipid include the following phospholipids, glycolipids, sterols, and saturated and unsaturated fatty acids.

<Phospholipids>

Examples of phospholipids include phosphatidylcholines (e.g., dioleoylphosphatidylcholine, dilauroylphosphatidylcholine, dimyristoylphosphatidylcholine, dipalmitoylphosphatidylcholine, and distearoylphosphatidylcholine), phosphatidylglycerols (e.g., dioleoylphosphatidylglycerol, dilauroylphosphatidylglycerol, dimyristoylphosphatidylglycerol, dipalmitoylphosphatidylglycerol, and distearoylphosphatidylglycerol), phosphatidyl ethanolamines (e.g., dilauroylphosphatidyl ethanolamine, dimyristoylphosphatidyl ethanolamine, dipalmitoylphosphatidyl ethanolamine, and distearoylphosphatidyl ethanolamine), phosphatidylserine, phosphatidylinositol, phosphatidic acid, cardiolipin, sphingomyelin, ceramide phosphorylethanolamine, ceramide phosphorylglycerol, ceramide phophorylglycerol phosphate, 1,2-dimyristoyl-1,2-deoxyphosphatidylcholine, plasmalogen, egg-yolk lecithin, soybean lecithin, hydrogenation products thereof, and the like.

<Glycolipids>

Examples of glycolipids include glyceroglycolipids (e.g., sulfoxyribosylglyceride, diglycosyldiglyceride, digalactosyldiglyceride, galactosyldiglyceride, and glycosyldiglyceride), sphingoglycolipids (e.g., galactosylcerebroside, lactosylcerebroside, and ganglioside), and the like.

<Sterols>

Examples of sterols include animal-derived sterols (e.g., cholesterol, cholesterol succinic acid, cholestanol, lanosterol, dihydrolanosterol, desmosterol, and dihydrocholesterol), plant-derived sterols (phytosterols) (e.g., stigmasterol, sitosterol, campesterol, and brassicasterol), microbially-derived sterols (e.g., zymosterol and ergosterol), and the like.

<Saturated and unsaturated fatty acids>

Examples of saturated and unsaturated fatty acids include saturated and unsaturated fatty acids having 12 to 20 carbon atoms, such as palmitic acid, oleic acid, stearic acid, arachidonic acid, myristic acid, and the like.

A membrane stabilizer is a component optionally added to the lipid membrane to physically or chemically stabilize the lipid membrane or to control the fluidity of the lipid membrane. The amount of the membrane stabilizer contained in the lipid membrane is usually 30% (mole ratio) or less, preferably 25% (mole ratio) or less, more preferably 20% (mole ratio) or less of the total amount of substances constituting the lipid membrane. It is to be noted that the lower limit value of the amount of the membrane stabilizer contained in the lipid membrane is 0.

Examples of the membrane stabilizer include sterols, glycerin and fatty acid esters thereof, and the like. Specific examples of the sterols include the same sterols as mentioned above. Examples of the glycerin fatty acid esters include triolein, trioctanoin, and the like.

An antioxidant is a component optionally added to the lipid membrane to prevent oxidation of the lipid membrane. The amount of the antioxidant contained in the lipid membrane is usually 30% (mole ratio) or less, preferably 25% (mole ratio) or less, more preferably 20% (mole ratio) or less of the total amount of substances constituting the lipid membrane. It is to be noted that the lower limit value of the amount of the antioxidant contained in the lipid membrane is 0.

Examples of the antioxidant include tocopherol, propyl gallate, ascorbyl palmitate, butylated hydroxytoluene, and the like.

A charged substance is a component optionally added to the lipid membrane to give a positive electric charge or a negative electric charge to the lipid membrane. The amount of the charged substance contained in the lipid membrane is usually 30% (mole ratio) or less, preferably 25% (mole ratio) or less, more preferably 20% (mole ratio) or less of the total amount of substances constituting the lipid membrane. It is to be noted that the lower limit value of the amount of the charged substance contained in the lipid membrane is 0.

Examples of the charged substance for giving a positive electric charge to the lipid membrane include: saturated and unsaturated aliphatic amines such as stearylamine, oleylamine, and the like; saturated and unsaturated cationic synthetic lipids such as dioleoyltrimethylammoniumpropane and the like; and the like. Examples of the charged substance for giving a negative electric charge to the lipid membrane include dicetylphosphate, cholesteryl hemisuccinate, phosphatidylserine, phosphatidylinositol, and phosphatidic acid.

A membrane protein is a component optionally added to the lipid membrane to maintain the structure of the lipid membrane and give functionality to the lipid membrane. The amount of the membrane protein contained in the lipid membrane is usually 10% (mole ratio) or less, preferably 5% (mole ratio) or less, more preferably 2% (mole ratio) or less of the total amount of substances constituting the lipid membrane. It is to be noted that the lower limit value of the amount of the membrane protein contained in the lipid membrane is 0.

Examples of the membrane protein include peripheral membrane proteins, integral membrane proteins, and the like.

As the lipid constituting the lipid membrane of the lipid membrane structure, for example, a lipid derivative having blood retentive function, temperature change-sensitive function, pH-sensitive function, or the like can be used. By doing so, it is possible to give one or two or more of these functions to the lipid membrane structure. By giving blood retentive function to the lipid membrane structure, it is possible to improve the ability of the lipid membrane structure to remain in the blood, thereby reducing the rate of capture of the lipid membrane structures by reticuloendothelial systems such as liver, spleen, and the like. Further, by giving temperature change-sensitive function and/or pH-sensitive function to the lipid membrane structure, it is possible to enhance the releasability of a target substance retained in the lipid membrane structure.

Examples of the blood retentive lipid derivative which can give blood retentive function to the lipid membrane structure include glycophorin, ganglioside GM1, phosphatidylinositol, ganglioside GM3, glucuronic acid derivatives, glutamic acid derivatives, polyglycerin phospholipid derivatives, and polyethylene glycol derivatives such as N-{carbonyl-methoxypolyethylene glycol-2000}-1,2-dipalmitoyl-sn-glycero-3-phosphoethanolami ne, N-{carbonyl-methoxypolyethylene glycol-5000}-1,2-dipalmitoyl-sn-glycero-3-phosphoethanolami ne, N-{carbonyl-methoxypolyethylene glycol-750}-1,2-distearoyl-sn-glycero-3-phosphoethanolamine, N-{carbonyl-methoxypolyethylene glycol-2000}-1,2-distearoyl-sn-glycero-3-phosphoethanolamin e, N-{carbonyl-methoxypolyethylene glycol-5000}-1,2-distearoyl-sn-glycero-3-phosphoethanolamin e, and the like.

Examples of the temperature change-sensitive lipid derivative which can give temperature change sensitive function to the lipid membrane structure include dipalmitoylphosphatidylcholine and the like. Examples of the pH-sensitive lipid derivative which can give pH-sensitive function to the lipid membrane structure include dioleoylphosphatidyl ethanolamine and the like.

The lipid membrane structure can have an antibody which can specifically recognize a cell containing a target nucleus, an enzyme secreted by the cell, or the like. Such an antibody is preferably a monoclonal antibody. The monoclonal antibody may be one kind of monoclonal antibody having specificity for a single epitope or a combination of two or more kinds of monoclonal antibodies having specificity for various epitopes. Further, the antibody may be either a monovalent antibody or a multivalent antibody, and a naturally occurring type (intact) molecule or a fragment or derivative thereof may be used. For example, $F(ab')_2$, Fab', Fab, a chimeric antibody or hybrid antibody having at least two antigen- or epitope-binding sites, a bispecific recombinant antibody such as quadrome or triome, an interspecific hybrid antibody, an anti-idiotypic antibody, or a derivative thereof obtained by chemically modifying or processing any one of these antibodies may be used. Further, an antibody synthetically or semisynthetically obtained using known cell fusion techniques, known hybridoma techniques, or antibody engineering techniques, an antibody obtained by DNA recombinant techniques using conventional techniques known from the viewpoint of producing antibodies, an antibody having a neutralization or binding property for a target epitope, or the like may be used.

The first and second lipid membrane contains an anionic lipid as a component thereof. The amount of the anionic lipid contained in the first lipid membrane is not particularly limited, but is usually 20 to 80% (mole ratio), preferably 40 to 60% (mole ratio), more preferably 45 to 55% (mole ratio) of the total amount of lipids contained in the first lipid membrane. By setting the amount of the anionic lipid contained in the first lipid membrane to a value within the above range, it is possible to improve the ability of the first lipid membrane to bind to and fuse with a nuclear membrane. The amount of the anionic lipid contained in the second lipid membrane is not particularly limited, but is usually 10 to 90% (mole ratio), preferably 10 to 50% (mole ratio), more preferably 10 to 30% (mole ratio) of the total amount of lipids contained in the second lipid membrane. By setting the amount of the anionic lipid contained in the second lipid membrane to a value within the above range, it is possible to improve the ability of the second lipid membrane to bind to and fuse with an endosomal membrane or a macropinosomal membrane.

The anionic lipid contained in the first lipid membrane is selected from cholesteryl hemisuccinate, phosphatidic acid, or cardiolipin, more preferably phosphatidic acid or cardiolipin. The anionic lipid contained in the second lipid membrane is selected from phosphatidic acid, cardiolipin, dialkyl phosphate, and diacyl phosphate.

In a case where the anionic lipid contained in the first lipid membrane is phosphatidic acid or cardiolipin, the amount of the anionic lipid contained in the first lipid membrane is preferably 40 to 60% (mole ratio) (particularly preferably, 45 to 55% (mole ratio)) of the total amount of lipids contained in the first lipid membrane, thereby making it possible to significantly improve the ability of the first lipid membrane to bind to and fuse with a nuclear membrane. In a case where the anionic lipid contained in the second lipid membrane is phosphatidic acid, cardiolipin, dialkyl phosphate, or diacyl phosphate, the amount of the anionic lipid contained in the second lipid membrane is preferably 10 to 50% (mole ratio) (particularly preferably, 10 to 30% (mole ratio)) of the total amount of lipids contained in the second lipid membrane, thereby making it possible to significantly improve the ability of the second lipid membrane to bind to and fuse with an endosomal membrane or a macropinosomal membrane.

The first or second lipid membrane preferably contains dioleoylphosphatidyl ethanolamine. By allowing the first lipid membrane to contain dioleoylphosphatidyl ethanolamine, it is possible to improve the ability of the first lipid membrane to bind to and fuse with a nuclear membrane. By allowing the second lipid membrane to contain dioleoylphosphatidyl ethanolamine, it is possible to improve the ability of the second lipid membrane to bind to and fuse with an endosomal membrane or a macropinosomal membrane. The amount of dioleoylphosphatidyl ethanolamine contained in the first lipid membrane is not particularly limited, but is usually 20 to 80% (mole ratio), preferably 40 to 60% (mole ratio), more preferably 45 to 55% (mole ratio) of the total amount of lipids contained in the first lipid membrane. By setting the amount of dioleoylphosphatidyl ethanolamine contained in the first lipid membrane to a value within the above range, it is possible to improve the ability of the first lipid membrane to bind to and fuse with a nuclear membrane. The amount of dioleoylphosphatidyl ethanolamine contained in the second lipid membrane is not particularly limited, but is usually 10 to 90% (mole ratio), preferably 50 to 90% (mole ratio), more preferably 70 to 90% (mole ratio) of the total amount of lipids contained in the second lipid membrane. By setting the amount of dioleoylphosphatidyl ethanolamine contained in the second lipid membrane to a value within the above range, it is possible to improve the ability of the second lipid membrane to bind to and fuse with an endosomal membrane or a macropinosomal membrane. It is to be noted that lipids are broadly classified into three types: cone-shaped type, cylinder-shaped type, and inverted cone-shaped type according to their proportion between polar groups and nonpolar groups contained therein. Among them, a cone-shaped lipid such as dioleoylphosphatidyl ethanolamine is a lipid in which hydrophobic groups occupy a larger volume than hydrophilic groups, and is also referred to as "nonbilayer lipid" (see, N. Oku, "Preparation and Experimentation of Liposome", pp. 27-33, Hirokawa-Shoten Ltd.). It is considered that a cone-shaped lipid has an inverted hexagonal structure in a lipid bilayer and therefore an inverted micelle structure is formed in the lipid bilayer, and the thus formed inverted micelle structure is involved in membrane fusion, membrane permeation, and the like.

The first and second lipid membrane have a membrane-permeable peptide. The membrane-permeable peptide is present in the first and second lipid membrane in a state where it can bind to a nuclear membrane. Preferably, the membrane-permeable peptide is present on the surface of the first or second lipid membrane. By allowing the membrane-permeable peptide to be present on the surface of the first lipid membrane, it is possible to improve the ability of the first lipid membrane to bind to and fuse with a nuclear membrane. By allowing the membrane-permeable peptide to be present on the surface of the second lipid membrane, it is possible to improve the ability of the second lipid membrane to bind to and fuse with an endosomal membrane or a macropinosomal membrane.

In a case where the membrane-permeable peptide is present on the surface of the first or second lipid membrane, the membrane-permeable peptide is present on at least the external surface of the first or second lipid membrane, that is, it is not always necessary for the membrane-permeable peptide to be present on the internal surface of the first or second lipid membrane. In a case where the first lipid membrane has a membrane-permeable peptide, the anionic lipid contained in the first lipid membrane is preferably cholesteryl hemisuccinate, phosphatidic acid, or phosphatidylserine.

By allowing the first lipid membrane to have a membrane-permeable peptide as well as such an anionic lipid, it is possible to improve the ability of the first lipid membrane to bind to and fuse with a nuclear membrane.

The membrane-permeable peptide contained in the first and second lipid membrane is not particularly limited as long as it can permeate a lipid membrane. Such a lipid membrane through which the membrane-permeable peptide can permeate is not particularly limited as long as it has a lipid bilayer structure, and examples thereof include biomembranes such as cell membranes and nuclear membranes and artificial membranes such as liposomal membranes. Examples of the membrane-permeable peptide include peptides having a protein transduction domain (PTD). Examples of the protein transduction domain include polyarginine, HIV-1-derived Tat (48-60) (GRKKRRQRRRPPQ), and HIV-1-derived Rev (34-50) (TRQARRNRRRRWRERQR). Such protein transduction domains are rich in arginine residues, and can therefore electrostatically interact with a negatively-charged nuclear membrane.

The total number of amino acid residues constituting the peptide having a protein transduction domain is not particularly limited, but is usually 4 to 40, preferably 6 to 30, more preferably 7 to 20. The total number of amino acid residues constituting the protein transduction domain is not particularly limited, but is usually 4 to 40, preferably 6 to 30, more preferably 7 to 20. The number of consecutive arginine residues constituting polyarginine as a protein transduction domain is usually 4 to 20, preferably 6 to 12, more preferably 7 to 10.

The peptide having a protein transduction domain may be composed of only a protein transduction domain, or may have any amino acid sequence added to the C-terminal and/or the N-terminal of a protein transduction domain. The amino acid sequence added to the C-terminal and/or the N-terminal of a protein transduction domain is preferably an amino acid sequence (e.g., polyproline) having rigidity. Unlike polyethylene glycol (PEG) which is soft and has no regular shape, polyproline is straight and maintains a certain level of rigidity. The amino acid residues contained in the amino acid sequence added to the C-terminal and/or the N-terminal of a protein transduction domain are preferably amino acid residues other than acidic amino acid residues. This is because there is a possibility that negatively-charged acidic amino acid residues electrostatically interact with positively-charged arginine residues and therefore the effect of the arginine residues contained in a protein transduction domain is reduced.

The amount of the membrane-permeable peptide present in the first lipid membrane is usually 2 to 20% (mole ratio), preferably 3 to 15% (mole ratio), more preferably 4 to 10% (mole ratio) of the total amount of lipids contained in the lipid membrane. By setting the amount of the membrane-permeable peptide present in the first lipid membrane to a value within the above range, it is possible to improve the ability of the first lipid membrane to bind to a nuclear membrane, thereby making it possible to efficiently bind the first lipid membrane to a nuclear membrane and therefore to efficiently induce membrane fusion between the first lipid membrane and the nuclear membrane.

The amount of the membrane-permeable peptide present in the second lipid membrane is usually 2 to 20% (mole ratio), preferably 3 to 15% (mole ratio), more preferably 4 to 10% (mole ratio) of the total amount of lipids contained in the lipid membrane. By setting the amount of the membrane-permeable peptide present in the second lipid membrane to a value within the above range, it is possible to improve the ability of the second lipid membrane to bind to an endosomal membrane or a macropinosomal membrane, thereby making it possible to efficiently bind the second lipid membrane to an endosomal membrane or a macropinosomal membrane and therefore to efficiently induce membrane fusion between the second lipid membrane and the endosomal membrane or the macropinosomal membrane.

An example of the aspect of the membrane-permeable peptide present in the first and second lipid membrane includes an aspect in which the membrane-permeable peptide binds to a lipid membrane constituent (e.g., a hydrophobic group, a hydrophobic compound), retained in the lipid membrane, so as to be partially or entirely exposed to the outside of the lipid membrane.

The lipid membrane constituent, to which the membrane-permeable peptide is to be bound, is not particularly limited, and examples thereof include: saturated and unsaturated fatty acid groups such as a stearyl group; cholesterol groups and derivatives thereof; phospholipids, glycolipids, and sterols; long-chain aliphatic alcohols (e.g., phosphatidyl ethanolamine, cholesterol); polyoxypropylene alkyls; and glycerin fatty acid esters. Among them, fatty acid groups having 10 to 20 carbon atoms (e.g., a palmitoyl group, an oleyl group, a stearyl group, an arachidoyl group) are particularly preferred.

The vector can retain a target substance to be delivered into a nucleus or a cell. For example, the vector can retain a target substance in an internal space (e.g., in a void formed in the lipid membrane structure), on the surface of a lipid membrane, in a lipid membrane, in a lipid membrane layer, or on the surface of a lipid membrane layer of the lipid membrane structure. In a case where the lipid membrane structure is a microparticle such as a liposome or the like, the target substance can be entrapped in the microparticle.

The type of target substance is not particularly limited, and examples of the target substance include drugs, nucleic acids, peptides, proteins, sugars, and complexes of two or more of them. The target substance can be appropriately selected according to the intended use such as diagnosis, treatment, or prevention of diseases. In a case where the lipid membrane structure retains a target substance intended to be used for diagnosis, treatment, or prevention of diseases, such a lipid membrane structure can be used as a constituent of a pharmaceutical composition. It is to be noted that "nucleic acids" include not only DNA and RNA but also analogues and derivatives thereof (e.g., peptide nucleic acids (PNAs), phosphorothioate DNA). The nucleic acid may be either single-stranded or double-stranded, and may be either linear or cyclic.

In a case where the target substance is a gene, the lipid membrane structure preferably has a compound having gene transfer function from the viewpoint of improving the efficiency of introducing a gene into a cell. Examples of such a compound having gene transfer function include O,O'-N-didodecanoyl-N-($\alpha$-trimethylammonioacetyl)-diethanol amine chloride, O,O'-N-ditetradecanoyl-N-($\alpha$-trimethylammonioacetyl)-dietha nolamine chloride, O,O'-N-dihexadecanoyl-N-($\alpha$-trimethylammonioacetyl)-diethan olamine chloride, O,O'-N-dioctadecenoyl-N-($\alpha$-trimethylammonioacetyl)-diethan olamine chloride, O,O',O''-tridecanoyl-N-($\omega$-trimethylammoniodecanoyl)aminome thane bromide, nor[$\alpha$-trimethylammonioacetyl]-didodecyl-D-glutamate, dimethyldioctadecylammonium bromide, 2,3-dioleyloxy-N-[2-(sperminecarboxamido) ethyl]-N,N-dimethy 1-1-propaneammonium trifluoroacetate, 1,2-dimyristyloxypropyl-3-dimethyl-hydroxyethylammonium bromide, and 3-$\beta$-[n-(N',N'-dimethylaminoethane)carbamoyl]cholesterol. These compounds having gene transfer function can be present in (bind to) an internal space (e.g., in a void formed in the lipid membrane structure), in a lipid membrane, on the surface of a lipid membrane, in a lipid membrane layer, or on the surface of a lipid membrane layer of the lipid membrane structure.

The target substance is preferably retained in the lipid membrane structure (particularly preferably, in the internal space of the lipid membrane structure) in the form of an aggregate of the target substance. This makes it possible to efficiently deliver the target substance into a nucleus.

The aggregate of the target substance may be composed of only the target substance or may contain a substance other than the target substance (e.g., a carrier for holding the target substance).

In a case where the target substance is positively charged, an aggregate of the target substance can be prepared by, for example, electrostatically binding the target substance to an anionic substance to form a complex. In a case where the target substance is negatively charged, an aggregate of the target substance can be prepared by, for example, electrostatically binding the target substance to a cationic substance to form a complex. In a case where the target substance is charged neither positively nor negatively, an aggregate of the target substance can be prepared by binding the target substance to a predetermined carrier in an appropriate manner (e.g., physical adsorption, hydrophobic bonding, chemical bonding) to form a complex. By adjusting the mixing ratio between the target substance and the cationic substance or the anionic substance in forming a complex, it is possible to prepare an aggregate of the target substance positively or negatively charged as a whole.

In a case where the target substance is nucleic acid, an aggregate of nucleic acid can be prepared by electrostatically binding nucleic acid to a cationic substance to form a complex. By adjusting the mixing ratio between the nucleic acid and the cationic substance in forming a complex, it is possible to prepare an aggregate of nucleic acid positively or negatively charged as a whole.

The cationic substance used for preparing an aggregate of the target substance is not particularly limited as long as it has a cationic group in its molecule. Examples of such a cationic substance include: cationic lipids (e.g., Lipofectamine (manufactured by Invitrogen)); cationic group-containing polymers; homopolymers and copolymers of basic amino acids such as polylysine, polyarginine, and copolymers of lysine and arginine, and derivatives thereof (e.g., stearylated derivatives); polycationic polymers such as polyethyleneimine, poly(allylamine), poly(diallyldimethylammonium chloride), and glucosamine; protamine and derivatives thereof (e.g., protamine sulfate); and chitosan. Among these cationic substances, stearylated polyarginine is particularly preferred. The number of arginine residues constituting polyarginine is usually 4 to 20, preferably 6 to 12, more preferably 7 to 10. The number of cationic groups contained in the cationic substance is not particularly limited, but is preferably 2 or more. The cationic group is not particularly limited as long as it can be positively charged, and examples thereof include an amino group, monoalkylamino groups such as a methylamino group and an ethylamino group; dialkylamino groups such as a dimethylamino group and a diethylamino group; an imino group; and a guanidino group.

The anionic substance used for preparing an aggregate of the target substance is not particularly limited as long as it has an anionic group in its molecule. Examples of such an anionic substance include: anionic lipids; anionic group-containing polymers; homopolymers and copolymers of acidic amino acids such as polyaspartic acid, and derivatives thereof; and polyanionic polymers such as xanthan gum, carboxyvinyl polymers, carboxymethyl cellulose polystyrene sulfonate, polysaccharide, and carrageenan. The number of anionic groups contained in the anionic substance is not particularly limited, but is preferably 2 or more. The anionic group is not particularly limited as long as it can be negatively charged, and examples thereof include functional groups having a terminal carboxyl group (e.g., a succinic acid residue, a malonic acid residue), a phosphate group, and a sulfate group.

The form of the lipid membrane structure is not particularly limited, and examples thereof include a form of dry mixture, a form of dispersion in an aqueous solvent, and a dried or frozen form thereof. These lipid membrane structures in such various forms can be produced using known methods such as hydration, ultrasonic treatment, ethanol injection, ether injection, reverse-phase evaporation, a surfactant method, freezing and thawing, and the like.

Hereinbelow, methods for producing lipid membrane structures in various forms will be described, but the form and production method of the lipid membrane structure according to the present invention are not limited thereto.

The lipid membrane structure in the form of dry mixture can be produced by, for example, once dissolving all the components of the lipid membrane structure in an organic solvent such as chloroform and then by subjecting the thus obtained solution to drying under reduced pressure using an evaporator or to spray drying using a spray drier.

The lipid membrane structure in the form of dispersion in an aqueous solvent can be produced by adding the lipid membrane structure in the form of dry mixture to an aqueous solvent and then emulsifying the thus obtained mixture using an emulsifier such as a homogenizer, an ultrasonic emulsifier, a high-pressure jet emulsifier, or the like. Alternatively, the lipid membrane structure in the form of dispersion in an aqueous solvent can also be produced by a method well-known as a method for producing a liposome, such as a reverse-phase evaporation method. In a case where it is desired that the size of the lipid membrane structure is controlled, extrusion can be performed under high pressure using a membrane filter or the like having pores uniform in pore size.

The composition of the aqueous solvent (dispersion medium) is not particularly limited, and examples of the aqueous solvent include: buffer solutions such as phosphate buffer, citrate buffer, and phosphate buffered saline; normal saline; culture media for cell culture; and the like. The lipid membrane structures can be stably dispersed in such an aqueous solvent (dispersion medium), but in order to further stably disperse the lipid membrane structures, a sugar or an aqueous solution thereof, a polyhydric alcohol or an aqueous solution thereof, or the like may be added to the aqueous solvent. Examples of the sugar include; monosaccharides (e.g. , glucose, galactose, mannose, fructose, inositol, ribose, and xylose); disaccharides (e.g., lactose, sucrose, cellobiose, trehalose, and maltose); trisaccharides (e.g., raffinose and melezitose); polysaccharides (e.g., cyclodextrin); sugar alcohols (e.g., erythritol, xylitol, sorbitol, mannitol, and maltitol); and the like. Examples of the polyhydric alcohol include glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, 1,3-butylene glycol, and the like. In order to stably store the lipid membrane structures dispersed in an aqueous solvent (dispersion medium) for a long period of time, it is preferred that electrolytes in the aqueous solvent (dispersion medium) are minimized from the viewpoint of physical stability (e.g., from the viewpoint of preventing aggregation). Further, from the viewpoint of chemical stability of lipid, it is preferred that the pH of the aqueous solvent (dispersion medium) is adjusted to lie within a range from weakly acidic to around neutral (pH 3.0 to 8.0) or dissolved oxygen is removed by nitrogen bubbling.

The lipid membrane structure in a dried or frozen form of the lipid membrane structure dispersed in an aqueous solvent can be produced by drying or freezing the lipid membrane structures dispersed in an aqueous solvent by a well-known method such as freeze drying or spray drying. In a case where the lipid membrane structures dispersed in an aqueous solvent are once produced and then dried, there is an advantage that it becomes possible to store the lipid membrane structures for a long period of time. In addition, in a case where an aqueous solution containing a medicinally active ingredient is added to the dried lipid membrane structures, there is also an advantage that it is possible to efficiently hydrate the lipid mixture and therefore to allow the lipid membrane structures to efficiently retain the medicinally active ingredient.

In a case where the lipid membrane structures dispersed in an aqueous solvent are dried by freeze drying or spray drying, a sugar or an aqueous solution thereof may be added to the aqueous solvent (dispersion medium) to stably store the lipid membrane structures for a long period of time. Examples of the sugar include: monosaccharides (e.g., glucose, galactose, mannose, fructose, inositol, ribose, and xylose); disaccharides (e.g., lactose, sucrose, cellobiose, trehalose, and maltose); trisaccharides (e.g., raffinose and melezitose); polysaccharides (e.g., cyclodextrin); sugar alcohols (e.g., erythritol, xylitol, sorbitol, mannitol, and maltitol); and the like. Further, in a case where the lipid membrane structures dispersed in an aqueous solvent are frozen, for example, the above-mentioned sugar or an aqueous solution thereof or a polyhydric alcohol or an aqueous solution thereof may be added to the aqueous solvent (dispersion medium) to stably store the lipid membrane structures for a long period of time. Examples of the polyhydric alcohol include glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, 1,3-butylene glycol, and the like. It is to be noted that both the sugar and the polyhydric alcohol may be added to the aqueous solvent (dispersion medium). The concentration of the sugar or the polyhydric alcohol in the aqueous solvent (dispersion medium) in which the lipid membrane structures are dispersed is not particularly limited, but the concentration of the sugar is preferably 2 to 20% (W/V), more preferably 5 to 10% (W/V), and the concentration of the polyhydric alcohol is preferably 1 to 5% (W/V), more preferably 2 to 2.5% (W/V). In a case where a buffer solution is used as the aqueous solvent (dispersion medium), the concentration of a buffering agent in the buffer solution is preferably 5 to 50 mM, more preferably 10 to 20 mM. The concentration of the lipid membrane structures in the aqueous solvent (dispersion medium) is not particularly limited. However, when expressed as a concentration of the total amount of lipids contained in the lipid membrane structures, the concentration of the lipid membrane structures in the aqueous solvent is preferably 0.1 to 500 mM, more preferably 1 to 100 mM.

The lipid membrane structure retaining an antibody can be produced by producing a lipid membrane structure and then adding an antibody to bind the antibody to the surface of a lipid membrane of the lipid membrane structure. Alternatively, the lipid membrane structure retaining an antibody can also be produced by producing a lipid membrane structure and then adding an antibody and a lipid derivative which can react with a mercapto group contained in the antibody to bind the antibody to the surface of a lipid membrane of the lipid membrane structure.

The form of a pharmaceutical composition containing the lipid membrane structures retaining a target substance is not particularly limited, and examples thereof include a form of dry mixture, a form of dispersion in an aqueous solvent, and a dried or frozen form thereof, and the like. These pharmaceutical compositions in such various forms can be produced in the same manner as the above-described lipid membrane structures in various forms.

The pharmaceutical composition in the form of dry mixture can be produced by, for example, once dissolving the components of the lipid membrane structure and a target substance in an organic solvent such as chloroform to obtain a mixture and then subjecting the mixture to drying under reduced pressure using an evaporator or to spray drying using a spray drier.

The pharmaceutical composition in a form that the lipid membrane structures retaining a target substance are dispersed in an aqueous solvent can be produced by any one of the following various known methods, which can be appropriately selected according to the form of a target substance retained in the lipid membrane structure, properties of a mixture, etc.

<Production Method 1>

A production method 1 is a method for producing a pharmaceutical composition in a form that the lipid membrane structures retaining a target substance are dispersed in an aqueous solvent, which includes once dissolving the components of the lipid membrane structure and a target substance in an organic solvent such as chloroform to obtain a mixture, subjecting the mixture to drying under reduced pressure using an evaporator or to spray drying using a spray drier to produce a dry mixture containing the lipid membrane structures and the target substance, and adding an aqueous solvent to the dry mixture to carry out emulsification using an emulsifier such as a homogenizer, an ultrasonic emulsifier, a high-pressure jet emulsifier, or the like. In a case where it is desired that the size (particle size) of the lipid membrane structure is controlled, extrusion can be performed under high pressure using a membrane filter having pores uniform in pore size. In the case of using the production method 1, it is necessary to dissolve the components of the lipid membrane structure and a target substance in an organic solvent to produce a dry mixture containing the lipid membrane structures and the target substance, but there is an advantage that it is possible to make the most of the interaction between the components of the lipid membrane structure and the target substance. That is, the production method 1 has an advantage that even in a case where the lipid membrane structure has a layered structure, the target substance can enter the inside of multiple layers of the layered structure and therefore the retention ratio of the target substance in the lipid membrane structure can be improved.

<Production Method 2>

A production method 2 is a method for producing a pharmaceutical composition in a form that the lipid membrane structures retaining a target substance are dispersed in an aqueous solvent, which includes once dissolving the components of the lipid membrane structure in an organic solvent, removing the organic solvent by evaporation to obtain dried matter, and adding an aqueous solvent containing a target substance to the dried matter to carry out emulsification. In a case where it is desired that the size (particle size) of the lipid membrane structure is controlled, extrusion can be performed under high pressure using a membrane filter having pores uniform in pore size. The production method 2 can be used for a target substance which is hard to be dissolved in an organic solvent but can be dissolved in an aqueous solvent. Further, the production method 2 has an advantage that when the lipid membrane structure is a liposome, the target substance can be retained also in the internal aqueous portion of the liposome.

<Production Method 3>

A production method 3 is a method for producing a pharmaceutical composition in a form that the lipid membrane structures retaining a target substance are dispersed in an aqueous solvent, which includes further adding an aqueous solvent containing a target substance to lipid membrane structures such as liposomes, emulsions, micelles, layered structures or the like dispersed in an aqueous solvent. The production method 3 can be used for a water-soluble target substance. As described above, the production method 3 is a method in which a target substance is externally added to lipid membrane structures which have been already produced, and therefore there is a possibility that when the target substance is a polymer, the target substance cannot enter the inside of the lipid membrane structures so that the target substance exists on (binds to) the surface of the lipid membrane structures. It is known that in a case where liposomes are used as the lipid membrane structures, use of the production method 3 results in the formation of a sandwich structure (generally referred to as a "complex") in which a target substance is sandwiched between liposome particles. In the case of using the production method 3, an aqueous dispersion liquid, in which only lipid membrane structures are dispersed,

is previously prepared, and therefore it is not necessary to take decomposition of a target substance during emulsification into consideration and it is easy to control the size (particle size) of the lipid membrane structure. Therefore, as compared to the production methods 1 and 2, the production method 3 makes it possible to more easily produce a pharmaceutical composition in a form in which the lipid membrane structures retaining a target substance are dispersed in an aqueous solvent.

<Production Method 4>

A production method 4 is a method for producing a pharmaceutical composition in a form that lipid membrane structures retaining a target substance are dispersed in an aqueous solvent, which includes adding an aqueous solvent containing a target substance to a dried product obtained by drying lipid membrane structures dispersed in an aqueous solvent. As in the case of the production method 3, the production method 4 can be used for a water-soluble target substance. A difference between the production method 3 and the production method 4 is a mode of presence of the lipid membrane structures and the target substance. According to the production method 4, lipid membrane structures dispersed in an aqueous solvent are first produced and then dried to produce a dried product, and therefore at this stage, the lipid membrane structures are present in a solid state as fragments of lipid membranes. In order to allow the fragments of lipid membranes to be present in a solid state, as descried above, a solvent obtained by adding a sugar or an aqueous solution thereof to an aqueous solvent is preferably used, and a solvent obtained by adding sucrose or an aqueous solution thereof to an aqueous solvent or a solvent obtained by adding lactose or an aqueous solution thereof to an aqueous solvent is more preferably used. When an aqueous solvent containing a target substance is added, water enters the fragments of lipid membranes present in a solid state to quickly hydrate them, and then the lipid membrane structures are reconstructed. As a result, lipid membrane structures retaining the target substance therein are produced.

According to the production method 4, an aqueous dispersion in which only the lipid membrane structures are dispersed is previously prepared, and therefore decomposition of the target substance during emulsification need not be taken into consideration and the size (particle size) of the lipid membrane structure can be easily controlled. For this reason, as compared to the production methods 1 and 2, the production method 4 makes it possible to more easily produce a pharmaceutical composition in a form that lipid membrane structures retaining a target substance are dispersed in an aqueous solvent. In addition, the production method 4 also has advantages that storage stability of an obtained pharmaceutical preparation (pharmaceutical composition) can be easily secured because freeze drying or spray drying is once carried out; lipid membrane structures having their original size (particle size) can be obtained even when the dried pharmaceutical preparation is rehydrated with an aqueous solution of a target substance; and a target substance can be easily retained inside the lipid membrane structures even when the target substance is a polymer. In a case where the target substance is a polymer, the use of the production method 3 makes a possibility that the target substance cannot enter the inside of the lipid membrane structures and therefore binds to the surface of the lipid membrane structures. The production method 4 is significantly different from the production method 3 in this point.

As another method for producing a pharmaceutical composition in a form that lipid membrane structures retaining a target substance are dispersed in an aqueous solvent, a method well known as a method for producing a liposome, for example, a reverse-phase evaporation method can be used. In a case where it is desired that the size (particle size) of the lipid membrane structure is controlled, extrusion can be performed under high pressure using a membrane filter having pores uniform in pore size.

Examples of a method for drying a dispersion liquid, in which lipid membrane structures retaining a target substance are dispersed in an aqueous solvent, include freeze drying and spray drying. As the aqueous solvent, a solvent obtained by adding the above-mentioned sugar or an aqueous solution thereof to an aqueous solvent is preferably used, and a solvent obtained by adding sucrose or an aqueous solution thereof to an aqueous solvent or a solvent obtained by adding lactose or an aqueous solution thereof to an aqueous solvent is more preferably used. An example of a method for freezing a dispersion liquid, in which lipid membrane structures retaining a target substance are dispersed in an aqueous solvent, includes a freezing method usually used. As the aqueous solvent, a solvent obtained by adding the above-mentioned sugar or an aqueous solution thereof to an aqueous solvent or a solvent obtained by adding the above-mentioned polyhydric alcohol or an aqueous solution thereof to an aqueous solvent is preferably used.

When the first lipid membrane of the vector is bound to a nuclear membrane, membrane fusion between the first lipid membrane and the nuclear membrane is induced so that a target substance retained in the lipid membrane structure is released into a nucleus, and as a result the target substance is delivered into the nucleus. For this reason, the vector can be used as a vector for delivering a target substance into a nucleus.

When the vector is introduced into a cell as an endosomal fraction, a macropinosomal fraction, or the like via a pathway such as endocytosis, macropinocytosis, or the like and then the second lipid membrane of the second vector is bound to an endosomal membrane or a macropinosomal membrane, membrane fusion between the second lipid membrane and the endosomal membrane or the macropinosomal membrane is induced so that a target substance retained in the lipid membrane structure escapes from an endosome, a macropinosome, or the like and is then released into a cell, and as a result the target substance is delivered into the cell. For this reason, the second vector can be used as a vector for delivering a target substance into a cell.

When the vector is introduced into a cell as an endosomal fraction, a macropinosomal fraction, or the like via a pathway such as endocytosis, macropinocytosis, or the like and then the second lipid membrane of the vector is bound to an endosomal membrane or a macropinosomal membrane , membrane fusion between the second lipid membrane and the endosomal membrane or the macropinosomal membrane is induced so that a target substance retained in the lipid membrane structure escapes from an endosome, a macropinosome, or the like and is then released into a cell. Then, when the first lipid membrane of the vector is bound to a nuclear membrane, membrane fusion between the first lipid membrane and the nuclear membrane is induced so that the target substance retained in the lipid membrane structure is released into a nucleus, and as a result the target substance is delivered into the nucleus. For this reason, the vector can be used as a vector for delivering a target substance into a nucleus.

A nucleus as a target for the vector may be either one separated from a cell or one present in a cell. The kind of organism whose nucleus is a target for the

vector is not particularly limited, and examples of the organism include animals, plants, microorganisms, and the like. However, among these organisms, animals are preferred, and mammals are more preferred. Examples of the mammals include humans, monkeys, cows, sheep, goats, horses, pigs, rabbits, dogs, cats, rats, mice, guinea pigs, and the like. The kind of cell containing a nucleus as a target for the first or third vector is not particularly limited, and examples of such a cell include somatic cells, germ cells, stem cells, and cultured cells thereof.

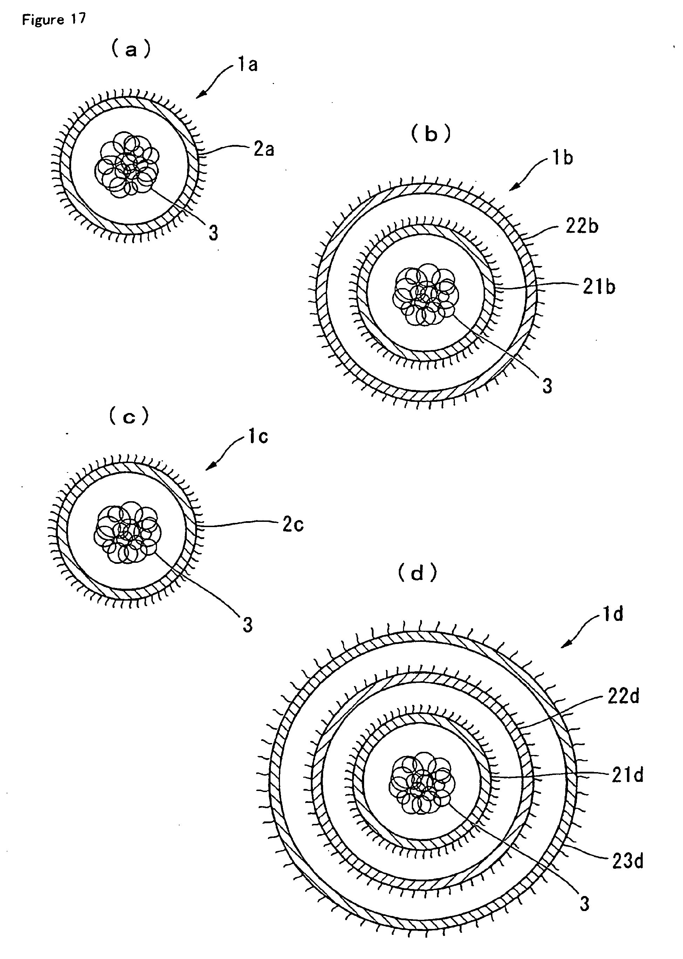

As shown in Fig. 17(a), a liposome as one embodiment of the vector is a unilamellar liposome 1a having a lipid membrane 2a and a target substance 3 entrapped inside the lipid membrane 2a. More specifically, in the unilamellar liposome 1a, the lipid membrane 2a serves as a first lipid membrane, and a membrane-permeable peptide containing polyarginine as a protein transduction domain (preferably, a membrane-permeable peptide made of polyarginine) is present on the surface of the lipid membrane 2a. The unilamellar liposome 1a can be transferred from the outside to the inside of a cell by means of the membrane permeable peptide present on the surface of the lipid membrane 2a with its original form being kept intact. When the lipid membrane 2a of the unilamellar liposome 1a which has been transferred into the cell is bound to a nuclear membrane by means of the membrane-permeable peptide present on the surface of the lipid membrane 2a, membrane fusion between the lipid membrane 2a and the outer membrane of a nucleus is induced so that the target substance 3 entrapped inside the lipid membrane 2a is released into the nucleus.

As shown in Fig. 17(b), a liposome as another embodiment of the vector is a bilamellar liposome 1b having a lipid membrane 21b, a lipid membrane 22b provided outside the lipid membrane 21b, and a target substance 3 entrapped inside the lipid membrane 21b. More specifically, in the bilamellar liposome 1b, the lipid membranes 21b and 22b serve as first lipid membranes, and a membrane-permeable peptide containing polyarginine as a protein transduction domain (preferably a membrane-permeable peptide made of polyarginine) is present on the surface of the lipid membranes 21b and 22b. The bilamellar liposome 1b can be transferred from the outside to the inside of a cell by means of the membrane-permeable peptide present on the surface of the lipid membrane 22b with its original form being kept intact. Then, when the lipid membrane 22b of the bilamellar liposome 1b which has been transferred into the cell is bound to the outer membrane of a nucleus by means of the membrane-permeable peptide present on the surface of the lipid membrane 22b, membrane fusion between the lipid membrane 22b and the outer membrane of the nucleus is induced so that the liposome permeates the outer membrane of the nucleus. The liposome loses the lipid membrane 22b due to membrane fusion with the outer membrane of the nucleus, but the liposome still has the lipid membrane 21b even after permeation through the outer membrane of the nucleus. When the lipid membrane 21b of the liposome which has permeated the outer membrane of the nucleus is bound to the inner membrane of the nucleus by means of the membrane-permeable peptide present on the surface of the lipid membrane 21b, membrane fusion between the lipid membrane 21b and the inner membrane of the nucleus is induced so that the target substance 3 entrapped inside the lipid membrane 21b is released into the nucleus. It is to be noted that the membrane-permeable peptide present on the surface of the lipid membrane 21b or 22b can be omitted.

As shown in Fig. 17(c), a liposome as one embodiment of the vector is a unilamellar liposome 1c having a lipid membrane 2c and a target substance 3 entrapped inside the lipid membrane 2c. More specifically, in the unilamellar liposome 1c, the lipid membrane 2c serves as a second lipid membrane, and a membrane-permeable peptide containing polyarginine as a protein transduction domain (preferably a membrane-permeable peptide made of polyarginine) is present on the surface of the lipid membrane 2c. The unilamellar liposome 1c can be transferred from the outside to the inside of a cell by means of the membrane-permeable peptide present on the surface of the lipid membrane 2c with its original form being kept intact. When the lipid membrane 2c of the unilamellar liposome 1c which has been transferred into the cell is bound to an endosomal membrane or a macropinosomal membrane by means of the membrane-permeable peptide present on the surface of the lipid membrane 2c, membrane fusion between the lipid membrane 2c and the endosomal membrane or the macropinosomal membrane is induced so that the target substance 3 entrapped inside the lipid membrane 2c is released into the cell (cytoplasm).

As shown in Fig. 17(d), a liposome as one embodiment of the vector is a trilamellar liposome 1d having a lipid membrane 21d, a lipid membrane 22d provided outside the lipid membrane 21d, a lipid membrane 23d provided outside the lipid membrane 22d, and a target substance 3 entrapped inside the lipid membrane 21d. More specifically, in the trilamellar liposome 1d, the lipid membranes 21d and 22d serve as first lipid membranes, the lipid membrane 23d serves as a second lipid membrane, and a membrane-permeable peptide containing polyarginine as a protein transduction domain (preferably a membrane-permeable peptide made of polyarginine) is present on the surface of the lipid membranes 21d, 22d, and 23d. The trilamellar liposome 1d can be transferred from the outside to the inside of a cell by means of the membrane-permeable peptide present on the surface of the lipid membrane 23d with its original form being kept intact. When the lipid membrane 23d of the trilamellar liposome 1d which has been transferred into the cell is bound to an endosomal membrane or a macropinosomal membrane by means of the membrane-permeable peptide present on the surface of the lipid membrane 23d, membrane fusion between the lipid membrane 23d and the endosomal membrane or the macropinosomal membrane is induced so that the liposome escapes from an endosome or a macropinosome. The liposome 1d loses the lipid membrane 23d due to membrane fusion with the endosomal membrane or the macropinosomal membrane, but the liposome 1d still has the lipid membranes 21d and 22d even after escape from the endosome or the macropinosome. Then, when the lipid membrane 22d is bound to the outer membrane of a nucleus by means of the membrane-permeable peptide present on the surface of the lipid membrane 22d, membrane fusion between the lipid membrane 22d and the outer membrane of the nucleus is induced so that the liposome permeates the outer membrane of the nucleus. The liposome 1d loses the lipid membrane 22d due to membrane fusion with the outer membrane of the nucleus, but the liposome 1d still has the lipid membrane 21d even after permeation through the outer membrane of the nucleus. Then, when the lipid membrane 21d of the liposome 1d which has permeated the outer membrane of the nucleus is bound to the inner membrane of the nucleus by means of the membrane-permeable peptide present on the surface of the lipid membrane 21d, membrane fusion between the lipid membrane 21d and the inner membrane of the nucleus is induced so that the target substance 3 entrapped inside the lipid membrane 21d is released into the nucleus. It is to be noted that the membrane-permeable peptide present on the surface of the lipid membrane 21d or 22d can be omitted.

These vectors described above can be used both in vivo and in vitro. In a case where any one of these vectors is used in vivo, it can be administered by a parenteral route such as an intravenous, intraperitoneal, subcutaneous, or nasal route. The dose and number of times of administration can be appropriately controlled according to the kind or amount of target substance entrapped in the lipid membrane structure according to the present invention.

Examples

Abbreviations used in the present examples are as follows.

BCA: bovine serum albumin
Chol: Cholesterol
CHEMS: cholesteryl hemisuccinate
DMSO: dimethyl sulfoxide
DNA: deoxyribonucleic acid
DOPE: 1,2-dioleoyl-sn-glycero-3-phosphoethanolamine
DOTAP: 1,2-dioleoyl-3-trimethylammonium propane
DTT: dithiothreitol
D'MEM: dulbecco's modified eagle medium
EDTA: ethylenediamine N',N',N',N'-tetraacetic acid
EGTA: ethylene glycol-bis($\beta$-aminoethyl ether)-N',N',N',N'-tetraacetic acid
Em: Emission
EPC: egg yolk L-$\alpha$-phosphatidylcholine
Ex: Excitation
GFP: green fluorescence protein
HBSS: Hank's balanced salt solution
HEPES: 2-[4-(2-Hydroxeyethyl)-1-piperazinyl]ethanesulfonic acid
LB: luria-bertani's broth
NBD-DOPE: 1,2-dioleoyl-sn-glycero-3-phosphoethanolamine-N-(7-nitro-2-1,3-benzoxadiazol-4-yl)
NLS: nuclear localization signal
NP-40: nonionic detergent
PA: 3-sn-phosphatidic acid
PBS: phosphate-buffered saline
PC: phosphatidylcholine

PLL: Poly-L-Lysine
PS: 3-sn-phosphatidyl-L-serine
Rho-DOPE: 1,2-dioleoyl-sn-glycero-3-phosphoethanolamine-N-(lissamine rhodamine B sulfonyl)
STR-R8: stearylated octaarginine
SV40: Simian Virus 40
rpm: round per minute

Manufacturers of reagents and materials used for experiments are as follows.

BCA protein assay kit (PIERCE)
CHEMS (SIGMA)
Cardiolipin (SIGMA)
Cholesterol (AVANTI)
DGDG (SIGMA)
DOPE (AVANTI)
DOTAP (AVANTI)
D'MEM (GIBCO)
EPC (NOF Corporation)
FCS (GIBCO)
GM1 (prepared in our laboratory)
Galactocerebroside (SIGMA)
Glucocerebroside (SIGMA)
Lactocerebroside (SIGMA)
Luciferase Assay Substrate (Promega)
NBD-DOPE (AVANTI)
Octylglucoside (SIGMA)
PA (SIGMA)
PG (SIGMA)
PI (SIGMA)
PLL (SIGMA)
PS (SIGMA)
Protamine sulfate (CALBIOCHEM)
Reporter Lysis Buffer (Promega)
Rho-DOPE (AVANTI)
Rho-R8 (obtained from Dr. S. Futaki, Kyoto University)
pEGFPluc (prepared in our laboratory)

Some reagents were prepared in the following manner.

PBS: 4.9 g of Dalbecco PBS(-) (Nissui Pharmaceutical Co., Ltd.) was dissolved in 500 mL of DDW and was then autoclaved.
LB+kan: 10 g of Bacto tryptone (Wako Pure Chemical Industries), 5 g of Yeast extract (Wako Pure Chemical Industries), and 10 g of NaCl (SIGMA) were dissolved in DDW so that the total volume of the solution was 1 L, and the solution was autoclaved. Then, kanamysine was added to the solution so that the concentration of kanamysine was 20 mg/mL.
HEPES buffer (10 mM) : 119 mg of HEPES (Wako Pure Chemical Industries) was dissolved in 50 mL of DDW to obtain a solution, and the pH of the solution was adjusted to 7.4 with 1N NaOH. Then, the solution was sterilized by filtration.
HEPES buffer 5% glucose (10 mM, pH 5.5) : 119 mg of HEPES (Wako Pure Chemical Industries) and 2.5 g of D-glucose (katayama Chemical Industries) were dissolved in 50 mL of DDW to obtain a solution, and the pH of the solution was adjusted to 7.4 with 1N NaOH. Then, the solution was sterilized by filtration in a clean bench.
Import buffer ($\times$10) : 4.77 g of HEPES (Wako Pure Chemical Industries), 10.8g of KOAc (Wako), 0.25 g of NaOAc (Wako), 0.43 g of MgOAc (SIGMA), and 0.23 g of EGTA·4Na were dissolved in DDW to obtain a solution, and the pH of the solution was adjusted to 7.3 with KOH. Then, DDW was added to the solution so that the total volume of the solution was 100 mL. 200 mM DTT was added to the solution just before use to dilute the solution 10-fold, and the diluted solution was sterilized by filtration.
200 mM DTT: 154.25 mg of DTT (nacalai tesque) was dissolved in DTT so that the total volume of the solution was 5 mL.

IGEPAL buffer: 0.5% (W/V) NP-40 (SIGMA), 10 mM NaCl, 3 mM $MgCl_2$, and 10 mM Tris-HCl were mixed, and the pH of the mixture was adjusted to 7.4.

HBSS: 4.0g of NaCl (SIGMA), 0.2 g of KCl, 0.07 g of $CaCl_2$, 0.1g of $MgSO_4 \cdot 7H_2O$, $KH_2PO_4$, 0.5 g of glucose, and 0.18 g of $NaHCO_3$ were dissolved in DDW, and the pH of the solution was adjusted to 7.5 with NaOH. Then, DDW was added to the solution so that the total volume of the solution was 500 mL.

(Example 1) Screening of lipids to find those having ability to fuse with nuclear membranes

1. Establishment of method for isolating nuclei and experimental system for assaying ability of lipids to bind to nuclear membranes

1-1. Introduction

The ability of lipids to bind to nuclear membranes is an essential factor to evaluate their ability to fuse with nuclear membranes. Therefore, in the first step of screening of lipids to find those having the ability to fuse with nuclear membranes, the ability of lipids to bind to nuclear membranes was evaluated. Since it has been reported that lipids such as PA and PS are fused with nuclear membrane (R. Lawaczeck.: Intraction of negatively charged liposomes with nuclear membranes: adsorption, lipid mixing and lysis of the vesicles. Biochem. Biophys. Acta. 903 (1987) 123-131), attention was focused on charged lipids, especially negatively-charged lipids in the following examples. Further, since it has been reported that a sugar chain has an affinity for lectin present in a nucleus, lipids having a sugar chain in their structures were also subjected to the screening. Lipids present in the nuclear membrane were also subjected to the screening. Lipids contained in the nuclear membrane are as follows: Cardiolipin 4%, PE 13%, PC 55%, PI 10%, PS 3%, PA 2%, lysoglycerophospholipid 3%, and SM 0.5%.

1-2. Establishment of method for isolating nuclei

1-2-1. Cell culture

(1) Preparation of Hela cells

A stock of Hela cells was mixed with and dissolved in 20 mL of D'MEM (containing 10% inactivated FCS) previously placed in a 50 mL conical tube. The conical tube was centrifuged at 1000 rpm at 25°C for 5 minutes. Then, the supernatant was removed from the conical tube, and 10 mL of D'MEM was added to the conical tube to suspend the cells. The cells were placed in a 10 cm dish and were then cultured in a $CO_2$ incubator at 37°C.

(2) Subculture of cells

The D'MEM in the 10 cm dish was removed by an aspirator, and then the cells were washed with 5 mL of PBS (-). Then, the PBS(-) was removed, and 2 mL of PBS(-) and 200 $\mu$L of a 0.5% trypsin/5 $\mu$L EDTA solution were added to the dish, and the cells were incubated in a 5% $CO_2$ incubator at 37°C for 5 minutes. Then, the cells were peeled off from the bottom surface of the dish. 8 mL of D'MEM was added to the dish to suspend the cells, and the thus obtained cell suspension was transferred into a 50 mL conical tube and was then centrifuged at 1000 rpm at 25°C for 5 minutes. Then, the supernatant was removed from the conical tube, and 10 mL of D'MEM was added to the conical tube to suspend the cells. The number of cells contained in the cell suspension was counted, and the cell suspension was diluted with D'MEM to an appropriate concentration, and the cells were placed in a 10 cm dish. It is to be noted that in a case where cell culture was continued for two or more days until the next subculture, the D'MEM was replaced with fresh one every two days.

(3) Cell counting

The cells were suspended in D'MEM to prepare a cell suspension, and 10 $\mu$L of the cell suspension was placed in a 1.5 mL sample tube. Then, 10 $\mu$L of a 0.3% trypan blue solution was added to the cell suspension, and they were thoroughly mixed with a pipette. Then, 10 $\mu$L of the thus obtained solution was placed onto a hemocytometer to count the number of cells.

(4) Preparation of stock of cells

Seventy percent confluent cells were used. The D'MEM in the 10 cm dish was removed by an aspirator, and the cells were washed with 5 mL of PBS(-). Then, the PBS ( - ) was removed from the dish, and then 2 mL of PBS(-) and 200 $\mu$L of a 0.5% trypsin/5 $\mu$L EDTA solution were added to the dish, and the cells were incubated in a 5% $CO_2$ incubator at 37°C for 5 minutes. Then, the cells were peeled off from the bottom surface of the dish. 8 mL of D'MEM was added to the dish to suspend the cells, and the thus obtained cell suspension was transferred into a 50 mL conical tube and was then centrifuged at 1000 rpm at 25°C for 5 minutes. Then, the supernatant was removed from the conical tube, and then 10 mL of D'MEM was added to the conical tube to suspend the cells to obtain a cell suspension. The number of cells contained in the cell suspension was counted. The cell suspension was again centrifuged at 1000 rpm at 25°C for 5 minutes, and then the supernatant was removed. A solution obtained by mixing D'MEM and DMSO at a ratio of 9:1 was added to the cells so that the cell suspension had a desired cell concentration. The cell suspension was suspended and then dispensed in 1.5 mL sample tubes with cap so that the volume of the cell suspension per sample tube was 1 mL. Then, the sample tubes were placed in a container filled with isopropanol, and frozen in a freezer kept at -80°C for 4 or more hours, and then stored in a cell storage tank filled with liquid nitrogen.

1-2-2. Method for isolating nuclei

(1) Protocol ideas

(a) Protocol using IGEPAL buffer

$6 \times 10^5$ cells/dish of Hela cells were placed in a 6 cm dish one day before. IGEPAL buffer and PBS were returned to room temperature before use. The cells were washed with PBS(-) twice, and then 500 $\mu$L of PBS (-) was added to the cells. The cells were collected using a cell scraper and transferred into a 1.5 mL sample tube, and were then centrifuged at 1000 rpm at 4°C for 5 minutes. The supernatant was removed by an aspirator, and then 500 $\mu$L of IGEPAL buffer was added to the pellet, and the IGEPAL buffer and the pellet were mixed with a pipette. The thus obtained suspension was centrifuged at 1400 g at 4°C for 5 minutes. The supernatant was removed by an aspirator, and then 500 $\mu$L of IGEPAL buffer was again added to the pellet, and the pellet and the IGEPAL buffer were mixed with a pipette. The thus obtained suspension was centrifuged at 1400g at 4°C for 5 minutes. The thus obtained isolated nuclei were suspended in Import buffer.

(b) Protocol using HBSS (J. Hasbold.: Flow cytometric cell division tracking using nuclei. Cytometry 40 (2000) 230-237)

An appropriate number of Hela cells were placed in a 10 cm dish two days before. An HBSS/0.1% BSA solution was placed on ice before the experiment. The cells were washed with PBS(-)/0.1% BSA/0.1% $NaN_3$ twice, and then 500 $\mu$L of Pubs(-)/0.1% Boa/0.1% $NaN_3$ was added to the cells. The cells were collected using a cell scraper and transferred into a 1.5 mL sample tube, and were then centrifuged at 1000 rpm at 4°C for 5 minutes. The supernatant was removed by an aspirator, and then 100 $\mu$L of HBSS/0.1% BSA was added to the pellet and the thus obtained suspension was mixed by tapping the tube. Then, 100 $\mu$L of an HBSS/0.1% BSA/0.2% NP-40 solution was further added to the suspension, and the suspension was incubated on ice for 5 minutes. Then, 800 $\mu$L of HBSS/0.1% BSA was added to the suspension, and the suspension was centrifuged at 10,000 rpm at 4°C for 30 seconds. The supernatant was removed by an aspirator, and 1 mL of HBSS/0.02% NP-40 was added to the pellet to suspend the pellet. The thus obtained suspension was centrifuged at 10,000 rpm at 4°C for 30 seconds. The supernatant was removed by an aspirator, and then 1 mL of HBSS/0.02% NP-40 was again added to the pellet to suspend the pellet. The thus obtained suspension was centrifuged at 10,000 rpm at 4°C for 30 seconds. The supernatant was removed by an aspirator. The thus obtained isolated nuclei were suspended in Import buffer.

(c) Nuclear staining with Hoechst

10 $\mu$L of Hoechst (1 mg/1mL) diluted 50-fold with buffer and 10 $\mu$L of a sample obtained by diluting the suspension of isolated nuclei to an appropriate concentration were mixed, and the mixture was incubated at room temperature for 10 minutes. Then, the mixture was placed onto a hemocytometer used also for cell counting, and the number of nuclei was counted using a fluorescent microscope.

(2) Consideration

In the case of the protocol using IGEPAL buffer, nuclei were aggregated during operation and therefore the nuclei were not suspended in IGEPAL buffer at all. It can be considered that this was mainly caused by the following factors: NP-40 contained in IGEPAL buffer promoted aggregation of cells; and the centrifugal speed was too high. Based on the consideration, the conditions of the second centrifugation were changed from 1400g, 4°C, and 5 minutes to 2000 rpm, 4°C, and 5 minutes. However, after all, aggregation of nuclei occurred and a particular change was not observed. Based on the result, 500 $\mu$L of IGEPAL buffer to be added for the second time was changed to buffer not containing NP-40. As a result, aggregation of nuclei still occurred but the nuclei were suspended in the buffer to some extent. On the other hand, in the case of the protocol using HBSS, aggregation of nuclei did not occur and therefore nuclei were easily suspended in HBSS. $2 \times 10^6$ cells were placed in a dish one day before, and then nuclei were extracted from these cells and labeled with Hoechst to count the number of the nuclei. As a result, the number of the nuclei was $1.329 \times 10^6$. The extraction efficiency was calculated from the concentration of cells placed in a dish and was found to be 69.5%. Further, there is a possibility that a centrifugal speed of 10,000 rpm is too high and therefore not only nuclei but also other fractions are precipitated. In order to investigate the possibility, the conditions of centrifugation were changed to 1,000 rpm, 4°C, and 5 minutes, and as a result the resulting pellet was too loose. For this reason, the idea of changing the conditions of centrifugation was abandoned because there was a fear that not only the supernatant but also nuclei were removed by an aspirator. Based on the result, the conditions of centrifugation were changed to 1400g, 4°C, and 1 minute. As a result, the resulting pellet had an appropriate hardness and was easily suspended in buffer, and fractions other than nuclei were not observed by a microscope. Therefore, these conditions were employed in the following experiments. It is to be noted that the number of cells placed in a dish for subculture two days before was decided to be $2.5 \times 10^6$ cells/dish, and the number of cells placed in a dish for subculture three days before was decided to be $1.5 \times 10^6$ cells/dish.

(3) Determination of protocol for extracting nuclei

Hela cells of appropriate concentration were placed in a 10 cm dish two days before. An HBSS/0.1% BSA solution was placed on ice before experiment. The cells were washed with PBS(-)/0.1% BSA/0.1% $NaN_3$ twice, and then 500 $\mu$L of PBS(-)/0.1% BSA/0.1% $NaN_3$ was added to the cells. Then, the cells were collected using a cell scraper and transferred into a 1.5 mL sample tube, and were then centrifuged at 1000 rpm at 4°C for 5 minutes. The supernatant was removed by an aspirator, and then 100 $\mu$L of HBSS/0.1% BSA was added to the pellet, and the thus obtained suspension was mixed by tapping the tube. Then, 100 $\mu$L of an HBSS/0.1% BSA/0.2% NP-40 solution was further added to the suspension, and the suspension was incubated on ice for 5 minutes. Then, 800 $\mu$Lof HBSS/0.1% BSA was added to the suspension, and the suspension was centrifuged at 1400g at 4°C for 1 minute. The supernatant was removed by an aspirator, and then 1 mL of HBSS/0.02% NP-40 was added to the pellet to suspend the pellet. The thus obtained suspension was centrifuged at 1400g at 4°C for 1 minute. The supernatant was removed by an aspirator, and then 1 mL of HBSS/0.02% NP-40 was again added to the pellet to suspend the pellet. The thus obtained suspension was centrifuged at 1400g at 4°C for 1 minute. The supernatant was removed by an aspirator, and the thus obtained isolated nuclei were suspended in Import buffer.

1-3. Establishment of experimental system for assaying binding of liposomes to nuclear membranes (binding assay)

1-3-1. Preparation of membrane-labeled liposomes

(1) Lipid:Chol= 2:1

A 10 mM lipid stock and 10mM Chol were mixed in a mole ratio of 2:1 to obtain a mixture, and a $CHCl_3$ solution containing NBD-DOPE in an amount of 1 mol% of the total lipid concentration was added to the mixture. Then, $CHCl_3$ was further added to the mixture and the mixture was dried in an atmosphere of nitrogen gas to prepare lipid film. Import buffer as an internal aqueous phase was added to the lipid film so that the total lipid concentration became 2 mM to hydrate the lipid film. Then, ultrasonic treatment was carried out to prepare liposomes.

(2) Lipid:DOPE= 2:3

Liposomes were prepared in the same manner as in 1-3-1 (1) except that a 10 mM lipid stock and 10mM DOPE were mixed in a mole ratio of 2:1 to obtain a mixture, and a $CHCl_3$ solution containing NBD-DOPE in an amount of 1 mol% of the total lipid concentration was added thereto.

1-3-2. Experimental method of binding assay

Nuclei were isolated by the method described in paragraph 1-2-2 and were then stained with Hoechst to count the number of the nuclei. 20 $\mu$L of each Probe Lip was added to about $5.0 \times 10^5$ nuclei placed in a 1.5 mL sample tube, and Import buffer was added to the sample tube so that the total volume of a sample contained in the sample tube was 200 $\mu$L. Likewise, 20 $\mu$L of Probe Lip was added to 180 $\mu$L of a 0.5% Triton solution to prepare a sample for measuring the maximum fluorescence intensity of liposomes. These samples were incubated at 37°C for 1 hour and then centrifuged at 1400g at 4°C for 1 minute, and then each of the pellets was washed twice. Then, the pellet was suspended in 100 $\mu$L of a 0.5% Triton solution, and the fluorescence intensity of the suspension was measured at 530 nm. The binding rate of liposomes to nuclear membranes was calculated by the following formula: binding rate (%) = (fluorescence intensity of liposomes bound to nuclei)/(maximum fluorescence intensity of liposomes used) $\times$ 100. It is to be noted that the number of nuclei used was corrected to $5.0 \times 10^5$.

1-4. Screening of lipids to find those having ability to bind to nuclear membranes (experimental results)

1-4-1. Evaluation of influence of electric charge of lipids on their ability to bind to nuclear membranes

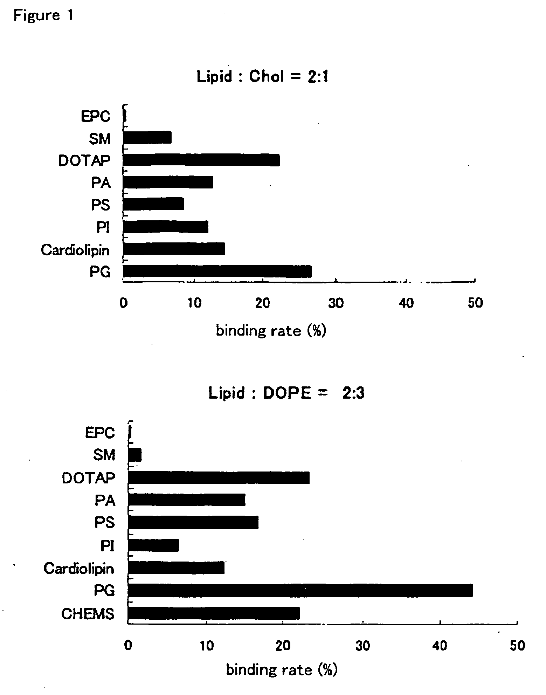

The influence of electric charge of lipids on their ability to bind to nuclear membranes was examined using the following lipids:EPC($\pm$), SM($\pm$), DOTAP(+), PA(-), PS(-), PI(-), Cardiolipin(-), PG(-), and CHEMS(-).
Various liposomes were prepared using each of these lipids and Chol or DOPE in combination, and then the binding rates of these liposomes to nuclear membranes were determined. The results are shown in Fig. 1. As can be seen from Fig. 1, liposomes using neutral lipids showed low binding rates, and particularly, a liposome using EPC showed a very low binding rate. On the other hand, a liposome using a positively-charged lipid showed a high binding rate. The reason for this can be considered as follows. The liposome using a positively-charged lipid electrostatically interacts with a nuclear membrane containing many negatively-charged lipids. Further, liposomes using negatively-charged lipids also showed high binding rates. Further, the binding rate of the liposome mainly containing Chol was not so different from that of the liposome mainly containing DOPE.

1-4-2. Evaluation of ability of other cell functional lipids to bind to nuclear membranes

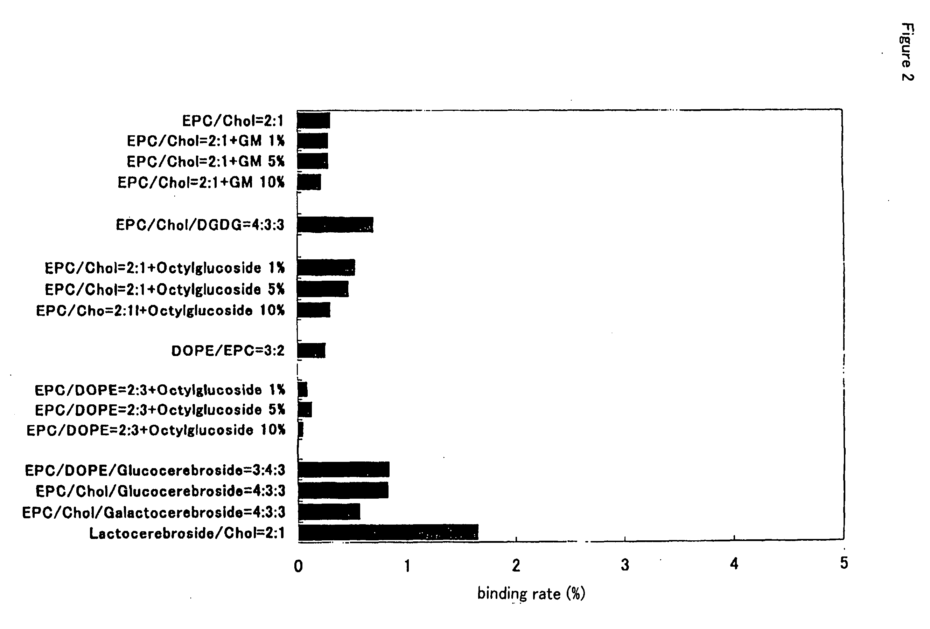

In order to evaluate the influence of factors other than electric charge on the ability of lipids to bind to nuclear membranes, an experiment was carried out using GM1 that is a lipid present in nuclear membranes and involved in physiological functions, cerebroside that is a lipid containing a sugar residue expected to interact with lectin present in a nucleus, etc. Lipids and surfactants used are as follows: GM1, DGDG, Galactocerebroside, Glucocerebroside, Lactocerebroside, and Octylglucoside.
Various liposomes were prepared using each of these lipids and EPC/Chol or EPC/DOPE in combination, and the binding rates of these liposomes to nuclear membranes were determined. The results are shown in Fig. 2. As can be seen from Fig. 2, all the liposomes showed very low binding rates. From the results, it can be considered that these lipids require not only an affinity for nuclei but also cytosolic factors to exhibit their physiological functions.

1-5. Summary

The above experiments were carried out to establish a method for isolating nuclei and an experimental method for assaying the ability of lipids to bind to nuclear membranes. The experimental results suggest that lipids need to have electric charge to bind to nuclear membranes of isolated nuclei. Further, it can be considered that the action of lipids involved in physiological functions in nuclear membranes and the interaction with sugar residues do not occur in test tubes containing only isolated nuclei because Importin, ATP, etc. involved in nuclear entry are not present in the test tubes. For this reason, electrically-charged lipids were used in the following experiments.

2. Establishment of experimental system for verifying ability of lipids to fuse with nuclear membranes using FRET

2-1. Introduction

FRET is a phenomenon observed during the interaction between the excitation wavelength of one of two fluorescent materials adjacent to each other and the fluorescence wavelength of the other fluorescent material. More specifically, FRET is a phenomenon in which, when two fluorescent materials are adjacent to each other and the wavelength of fluorescence emitted by exciting one of the two fluorescent materials (A) is close to the excitation wavelength of the other fluorescent material (B), the fluorescent material (B) is excited by the fluorescence wavelength to emit fluorescence and therefore it becomes impossible to observe the fluorescence of the fluorescent material (A). In this research, liposomes containing two kinds of fluorescent materials, NBD and Rho were prepared. When the liposomes are not fused with nuclear membranes, fluorescence energy derived from NBD is transferred to Rho, and therefore the fluorescence of NBD is suppressed but fluorescence with a wavelength of 590 nm that is the fluorescence wavelength of Rho is increased. On the other hand, when the liposomes are fused with nuclear membranes, the distance between the two fluorescent materials is increased so that the transfer of energy to Rho is canceled and therefore fluorescence with a wavelength of 530 nm that is the fluorescence wavelength of NBD is recovered. Based on the principles, fluorescence-labeled liposomes were prepared using the electrically-charged lipids which had been used in the experiments described in Chapter 1, and the ability of the liposomes to fuse with nuclear membranes was evaluated.

2-2. Verification of ability of lipids to fuse with nuclear membranes using FRET

2-2-1. Method for quantitatively determining the amount of phospholipid

In order to equalize the concentration of isolated nuclei and the concentration of liposomes, the amount of phospholipid contained in nuclei was measured using a kit, Phospholipid C-Test Wako according to the operation manuals included in the kit.

2-2-2. Examination of conditions of fluorescent lipids

In order to observe the cancellation of FRET most clearly, optimum conditions of fluorescent lipids were examined by changing the concentrations of the fluorescent lipids. Various Probe Lips were prepared by compositions as shown in Table 1.
[Table 1]

| NBD-DOPE | Rho-DOPE |
| --- | --- |
| 2 mol% | 1 mol% |
| 1.5 mol% | 0.5 mol% |
| 1 mol% | 0.5 mol% |

(1) Preparation of Non-probe Lips (pH 7.4 or pH 4.7)

6.65 mg (8.65 $\mu$mol) of EPC and 1.7 mg (4.35 $\mu$mol) of Chol were prepared and dissolved in 2.5 mL of $CHCl_3$ to obtain a solution. Then, the solution was stirred using a vortex mixer, and 1 mL of the solution was placed into each of two glass test tubes. The solution in the glass test tubes was dried in an atmosphere of nitrogen gas to prepare lipid film. Then, 1 mL of PBS(-) was added to each of the two glass test tubes to hydrate the lipid film for 10 minutes. Then, ultrasonic treatment was carried out to prepare liposomes. Then, the liposome suspension was diluted 2.5-fold with PBS (-) (pH 7.4 or 4.7) (final total lipid concentration: 2 mM).

(2) Preparation of Probe Lips

30 $\mu$L of 10 mM DOPE and 20 $\mu$L of 10 mM CHEMS were placed in a glass test tube (lipid concentration: 0.5 $\mu$mol), and then x mol% of NBD-DOPE and y mol% of Rho-DOPE (see Table 1) were added to the test tube, and then $CHCl_3$ was further added to the test tube. The thus obtained mixture was dried in an atmosphere of nitrogen gas to prepare lipid film. Then, 250 $\mu$L of PBS(-) (pH 7.4) was added to the lipid film to hydrate the lipid film for 10 minutes. Then, ultrasonic treatment was carried out to prepare liposomes.

(3) Analysis of FRET

In a 1.5 mL sample tube, the 2 mM Probe Lip and 2 mM Non-probe Lip prepared above were mixed in a ratio of 1:9 to prepare a sample. Further, the Probe Lip and PBS were mixed in a ratio of 1:9 to prepare a FRET control, and the Probe Lip and 0.5% Triton (pH 7.4) were mixed in a ratio of 1: 9 to prepare a control for measuring the maximum fluorescence intensity of liposomes. These samples were incubated at 37°C for 1 hour. Then, the pH of each of the samples was adjusted with 1N HCl, and the fluorescence intensity of each of the samples was measured at ex 470 nm and em 530 nm and 590 nm. The Transfer Efficiency of each sample was calculated by substituting the fluorescence intensities at 530 nm of the sample and controls into the following formula: TF = (F-F0)/(Fmax-F0) $\times$ 100, where TF represents Transfer Efficiency, F represents the fluorescence intensity of the sample, F0 represents the fluorescence intensity of the blank, and Fmax represents the fluorescence intensity of the control obtained by breaking the liposomes by Triton having a final concentration of 0.5%.
The results are shown in Table 2. DOPE/CHEMS are lipids which are pH-dependently fused with membranes, and therefore it can be considered that membrane fusion hardly occurs at pH 4.7 but occurs at pH 7.4. As can be seen from Table 2, the difference between TF at pH 4.7 and TF at pH 7.4 is the largest when NBD and Rho are added in a ratio of 1:0.5, and at this time, TF at pH 7.4 is the largest. From the result, it can be considered that the cancellation of FRET is most clearly observed under the condition. Based on the results, this concentration was employed, respectively, in the following experiments.

[Table 2]

| NBD : Rho | | 2 : 1 | 1.5 :0.5 | 1 : 0.5 |
|---|---|---|---|---|
| TF(%) | pH 7.4 | 6.35 | 11.9 | 0.69 |
| | pH 4.7 | 2.50 | 3.17 | 26.34 |

2-2-3. Preparation of membrane-labeled liposomes (1) Lipid:Chol =2:1

A 10 mM lipid stock and 10 mM Chol were mixed in a mole ratio of 2:1 to obtain a mixture, and then a $CHCl_3$ solution containing NBD-DOPE in an amount of 1 mol% of the total lipid concentration and a $CHCl_3$ solution containing Rho-DOPE in an amount of 0.5 mol% of the total lipid concentration were added thereto, and then $CHCl_3$ was further added thereto. The thus obtained mixture was dried in an atmosphere of nitrogen gas to prepare lipid film. Then, Import buffer as an inner aqueous phase was added to the lipid film so that the total lipid concentration became 2 mM to hydrate the lipid film. Then, ultrasonic treatment was carried out to prepare liposomes.

(2) Lipid:DOPE = 2:3

Liposomes were prepared in the same manner as in 2-2-3 (1) except that a 10 mM lipid stock and 10 mM DOPE were mixed in a mole ratio of 2:1, and then a $CHCl_3$ solution containing NBD-DOPE in an amount of 1 mol% of the total lipid concentration and a $CHCl_3$ solution containing Rho-DOPE in an amount of 0.5 mol% of the total lipid concentration were added thereto.

2-2-4. FRET

The amount of phospholipid contained in nuclei isolated by the method described in Section 1-2-2 was measured to determine the amount of PC contained in the nuclei. It has been reported that the amount of PC contained in the nuclear membrane is 55%. Based on the report, the total concentration of lipids in the nuclei was determined. Then, the suspension of the isolated nuclei was diluted to have the same total lipid concentration (2 mM) as the Probe Lip. The Probe Lip prepared by the method described in Section 2-2-3 and the 2 mM suspension of isolated nuclei were mixed in a ratio of Probe Lip:nuclei= 1:9 in a 1.5 mL sample tube, and the thus obtained mixture was incubated at 37°C for 1 hour. A control was prepared by mixing Probe Lip and Import buffer in a ratio of 1:9 and incubating the mixture at 37°C for 1 hour. A control for measuring the maximum fluorescence intensity of the liposomes was prepared by mixing Probe Lip and 0.5% Triton in a ratio of 1:9 and incubating the mixture at 37°C for 1 hour. After the completion of incubation, each sample was diluted to 0.1 mM to measure a fluorescence intensity (excitation wavelength: 470 nm, absorption wavelength: 530 nm, 590 nm). The fluorescence intensity of the 2 mM sample was determined by calculation based on the measured fluorescence intensity of the 0.1 mM sample, and TF was calculated by substituting the fluorescence intensities at 530 nm of the sample and controls into the formula shown in Section 2-2-2.

2-3 Screening of lipids to find those having ability to fuse with nuclear membranes (experimental results)

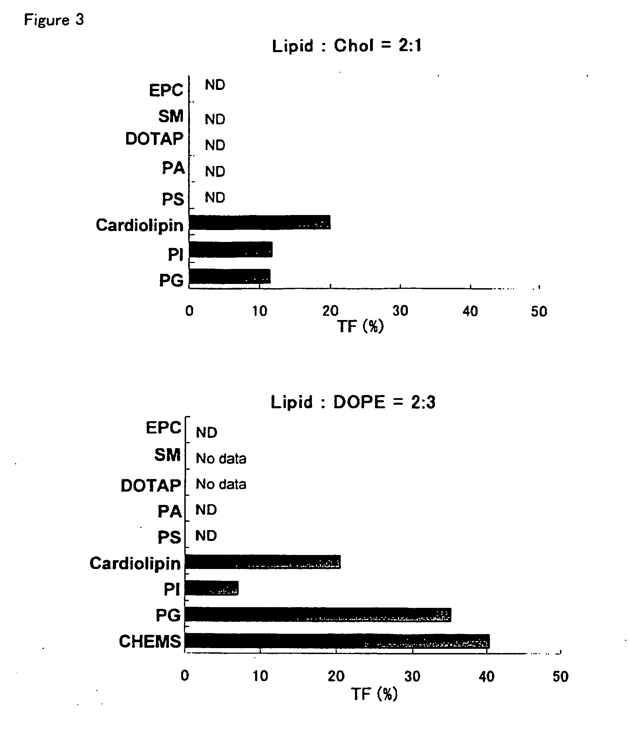

FRET analysis was performed using EPC and lipids which had been found to have ability to bind to nuclear membranes by the experiment results described in Section 1-4. Lipids used in this experiment are as follows: EPC($\pm$), SM($\pm$), DOTAP(+), PA(-), PS(-), PI(-), Cardiolipin(-), PG(-), and CHEMS(-).
Various liposomes were prepared using these lipids by the method described in Section 2-2-3, and TFs of these liposomes were measured. The results are shown in Fig. 3. As can be seen from Fig. 3, broadly speaking, liposomes mainly containing Chol showed low TFs and liposomes mainly containing DOPE showed relatively, high TFs. However, it is difficult to say that the experimental results of the liposomes mainly containing DOPE are good because many spectra could not be analyzed and some data could not be obtained. DOPE per se has a very unstable structure, and therefore the use of DOPE together with another lipid makes it easy to form an inverted hexagonal structure, thereby improving the ability of the liposomes to fuse with membranes. From the findings, it can be considered that some of the liposomes mainly containing DOPE were probably broken during incubation and therefore spectral analysis could not be well performed. On the other hand, it may be considered that the data of the liposomes mainly containing Chol are correct because spectra showing that cancellation of FRET had not occurred were well reproduced. When Chol is used together with a lipid, Chol functions as a liner of a lipid membrane so that the lipid membrane becomes hard. Therefore, it was predicted that the TFs of the liposomes mainly containing Chol would be decreased. However, among these various liposomes mainly containing Chol, a liposome mainly containing Cardiolipin, a liposome mainly containing PI, and a liposome mainly containing PG showed relatively high TFs even when these liposomes became hard due to the use of Chol. This is because it can be considered that Cardiolipin, PI, and PG per se have high ability to fuse with membranes. Further, it can be considered that an increase in the amount of DOPE among lipids constituting the liposomes had some effect on the ability of the liposomes to fuse with membranes so that their ability to fuse with membranes was reduced.

2-4. Summary

The above experiments were carried out to establish an experimental system for verifying the ability of lipids to fuse with nuclear membranes using FRET. From the experimental results, it has been confirmed that electrically-charged lipids not only bind to nuclear membranes but also fuse with nuclear membranes. However, it has been suggested that there is a possibility that liposomes are broken during incubation, and therefore it is necessary to devise a way to carry out incubation. Further, it can be considered that it is important to further study lipid composition such as a mixing ratio of DOPE or Chol to improve the ability of liposomes to fuse with membranes.

3. Evaluation of affinity between STR-R8-modified liposomes and nuclear membranes

3-1. Introduction

STR-R8 ($CH_3(CH_2)_{16}CONHRRRRRRRR$) is a very strongly basic peptide. It has been reported that a STR-R8-modified liposome is introduced into a cell by macropinocytosis and then delivered into a nucleus with its lipid membrane structure being kept intact (S. Futaki, "Membrane permeable basic peptide", PROTEIN, NUCLEIC ACID, AND ENZYME, Vol. 47(2002), 1415-1419). Further, by adding STR-R8 to a liposome solution, it is possible to easily modify liposomes with STR-R8 to allow the liposomes to have positive electric charge, thereby improving the ability of the liposomes to bind to nuclear membranes. Therefore, it can be considered that an effective gene vector can be constructed by using a lipid which can keep its ability to fuse with nuclear membranes even when it is modified with STR-R8 and which can have an improved ability to bind to nuclear membranes by modification with STR-R8. In order to examine the influence of modification of liposomes with STR-R8 on their affinity for nuclear membranes and the correlation between the ability of liposomes modified with STR-R8 to bind to nuclear membranes and their ability to fuse with nuclear membranes, the following experiments were performed. It is to be noted that liposomes were prepared by mixing a lipid and DOPE (Chol) in a ratio of 9:2 so that the liposomes were easily modified with STR-R8.

3-2. Evaluation of affinity between STR-R8-modified liposomes and nuclear membranes

3-2-1. Preparation of membrane-labeled liposomes

(1) Preparation of liposomes for binding assay

(a) Lipid:Chol= 9:2

A 1 mM lipid stock and a 1 mM Chol stock were mixed in a mole ratio of 9:2, and then a $CHCl_3$ solution containing NBD-DOPE in an amount of 1 mol% of the total lipid concentration was added thereto, and then $CHCl_3$ was further added thereto. The thus obtained mixture was dried in an atmosphere of nitrogen gas to prepare lipid film. Then, Import buffer as an inner aqueous phase was added to the lipid film so that the total lipid concentration became 0.55 mM to hydrate the lipid film. Then, ultrasonic treatment was carried out to prepare liposomes. In the case of modifying the liposomes with STR-R8, 5 mol% of STR-R8 was added to the liposome solution obtained by ultrasonic treatment, and then they were mixed with a pipette and the mixture was left stand at room temperature for 30 minutes.

(b) Lipid:DOPE = 2:9

Liposomes and STR-R8-modified liposomes were prepared in the same manner as in Section 3-2-1 (1)(a) except that a 1 mM lipid stock and a 1mM DOPE stock were mixed in a mole ratio of 2:9, and a $CHCl_3$ solution containing NBD-DOPE in an amount of 1 mol% of the total lipid concentration was added thereto.

(2) Preparation of liposomes used for FRET analysis Lipid:Chol= 9:2

A 1 mM lipid stock and a 1 mM Chol stock were mixed in a mole ratio of 9:2, and then a $CHCl_3$ solution containing NBD-DOPE in an amount of 1 mol% of the total lipid concentration and a $CHCl_3$ solution containing Rho-DOPE in an amount of 0.5 mol% of the total lipid concentration were added thereto, and then $CHCl_3$ was further added thereto. Then, the thus obtained mixture was dried in an atmosphere of nitrogen gas to prepare lipid film. Then, Import buffer as an internal aqueous phase was added to the lipid film so that the total lipid concentration became 0.55 mM to hydrate the lipid film. Then, ultrasonic treatment was carried out to prepare liposomes. In the case of modifying the liposomes with STR-R8, 5 mol% of STR-R8 was added to the liposome solution obtained by ultrasonic treatment, and then they were mixed with a pipette and the mixture was left stand at room temperature for 30 minutes.

(b) Lipid:DOPE= 2:9

Liposomes and STR-R8-modified liposomes were prepared in the same manner as in Section 3-2-1 (2)(a) except that a 1 mM lipid stock and a 1mM DOPE stock were mixed in a mole ratio of 2:9, and a $CHCl_3$ solution containing NBD-DOPE in an amount of 1 mol% of the total lipid concentration and a $CHCl_3$ solution containing Rho-DOPE in an amount of 0.5 mol% of the total lipid concentration were added thereto.

3-2-2. Binding assay

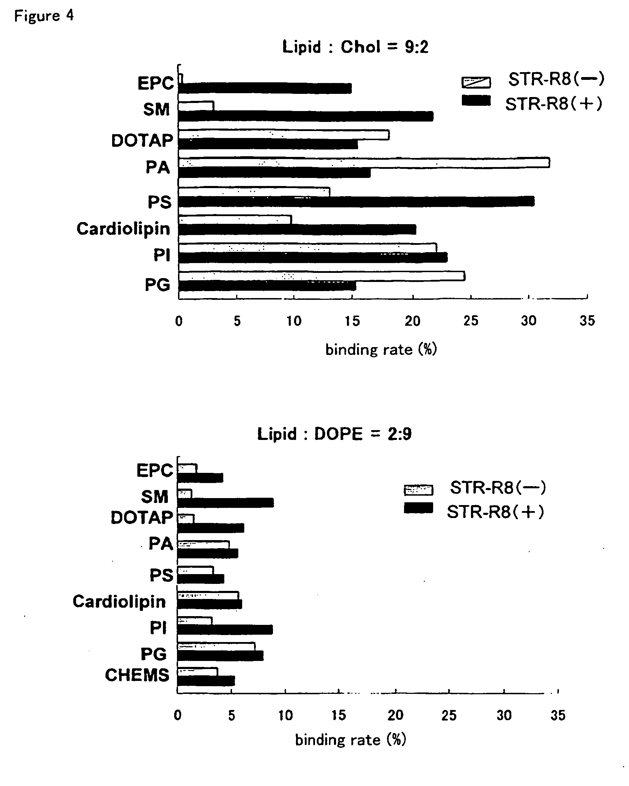

A binding assay was performed for liposomes (STR-R8 +/-) using lipids which had showed high binding rates in the experiment described in Section 1-4-1. The binding assay was performed by the method described in Section 1-3-2. The results are shown in Fig. 4. As shown in Fig. 4, almost all the lipids showed increased binding rates to nuclear membranes. It is to be noted that the binding rates of the liposomes mainly containing DOPE were significantly lower than those shown in Section 1-4-1. This is because these liposomes had a higher DOEP content than those used in the experiment described in Section 1-4-1, and therefore it can be considered that these liposomes mainly containing DOPE became difficult to bind to nuclear membranes because of some structural properties of DOPE. On the other hand, liposomes mainly containing Chol had a lower Chol content than those used in the experiment in Section 1-4-1, and therefore it can be considered that these liposomes mainly containing Chol became easy to bind to nuclear membranes because of their improved lipid membrane fluidity.

3-2-3. FRET analysis

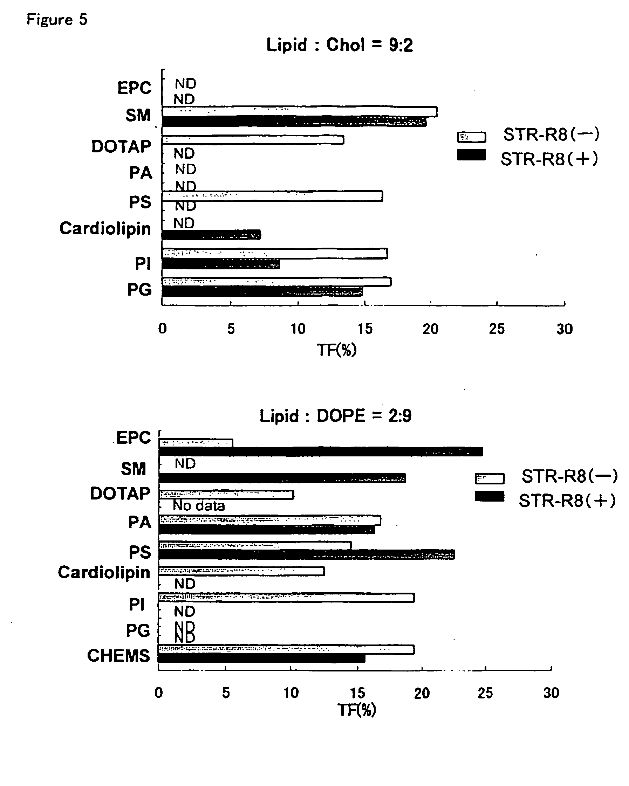

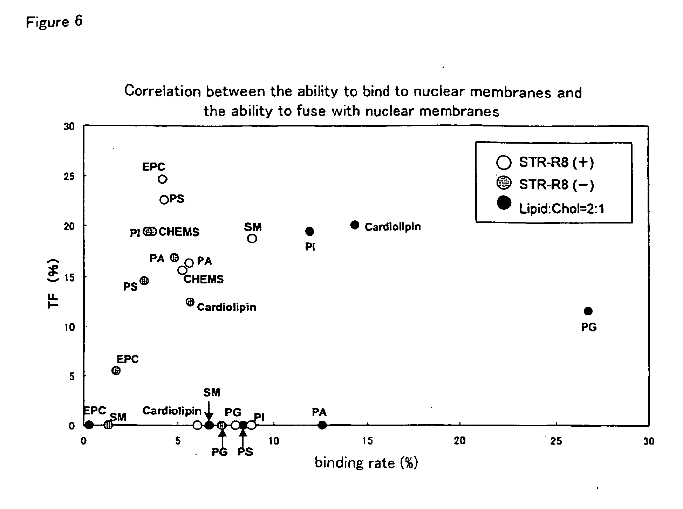

FRET analysis was performed for the same lipids used in the experiment described in Section 3-2-2 by the method described in Section 2-2-4. The results (TFs) are shown in Fig. 5.
As shown in Fig. 5, unlike the binding assay, not all the lipids modified with STR-R8 showed improved TFs. On the contrary, in some cases, cancellation of FRET was not observed at all. The ability of a liposome using DOPE/PG to fuse with nuclear membranes was significantly lower than that of the liposome prepared by mixing a lipid and DOPE in a ratio of 2:3. This result suggests that the ability of PG to fuse with nuclear membranes is lowered when it is used together with DOPE. However, the ability of liposome using DOPE/EPC or DOPE/SM to fuse with nuclear membranes was significantly improved by modifying them with STR-R8 in spite of the fact that these liposomes not modified with STR-R8 were hardly bound to and fused with nuclear membranes. From the result, it can be considered that these liposomes were electrically charged by modification with STR-R8 and therefore they became able to have an affinity for cells.
Then, analysis was performed to examine the correlation between the ability of liposomes to bind to nuclear membranes and their ability to fuse with nuclear membranes by using liposomes prepared by mixing a lipid and Chol in a ratio of 2: 1 which had showed a particularly low ability to fuse with nuclear membranes in the experiments described above, liposomes prepared by mixing a lipid and DOPE in a ratio of 2:9 which had showed a high ability to fuse with nuclear membranes in the experiments described above, and liposomes obtained by modifying the liposomes (lipid:DOPE= 2: 9) with STR-R8. The results are shown in Fig. 6. As shown in Fig 6, although there are some exceptions, broadly speaking, the lines of the DOPE liposomes have larger gradients than the lines of the Chol liposomes. Further, in Fig. 6, the lines of the DOPE liposomes modified with STR-R8 shift to the right side of the lines of the DOPE liposomes with their gradients being substantially maintained. This result suggests that the DOPE liposomes have a higher ability to fuse with nuclear membranes per unit binding amount than the Chol liposomes and that the ability of the DOPE liposomes to bind to nuclear membranes is improved by modification with STR-R8. From the results, it has become apparent that among the liposomes using negatively-charged lipids, the liposome using DOPE/CHEMS, the liposome using DOPE/PA, and the liposome using DOPE/PS have an especially excellent ability to fuse with nuclear membranes. In addition, it has also become apparent that the ability of the liposome using a neutral lipid such as EPC or SM to bind to and fuse with nuclear membranes is significantly improved by modification with STR-R8.

3-3. Visualization of affinity between STR-R8-modified liposomes and nuclear membranes

3-3-1. Preparation of membrane-labeled liposomes entrapping GFP

A 1 mM lipid stock and a 1 mM DOPE stock were mixed in a mole ratio of 9:2, and then a $CHCl_3$ solution containing Rho-DOPE in an amount of 0.5 mol% of the total lipid concentration was added thereto, and then $CHCl_3$ was further added thereto. The thus obtained mixture was dried in an atmosphere of nitrogen gas to prepare lipid film. Then, GFP was diluted with 10 mM HEPES to 0.125 mg/mL, and the HEPES solution of GFP was added as an internal aqueous phase to the lipid film so that the total lipid concentration became 0.55 mM to hydrate the lipid film. Then, ultrasonic treatment was carried out to prepare liposomes. Then, 5 mol% of STR-R8 was added to the liposome solution obtained by ultrasonic treatment, and they were mixed with a pipette and the mixture was left stand at room temperature for 30 minutes.

3-3-2. Analysis of affinity between liposomes and nuclear membrane by confocal laser microscope

(1) Operational method

An experiment was carried out using various liposomes having the following different lipid compositions. EPC/Chol/STR-R8, DOPE/CHEMS/STR-R8, DOPE/PA/STR-R8, DOPE/PS/STR-R8

These liposomes were prepared by the method described in Section 3-3-1. Nuclei were isolated by the method described in Section 1-2-2, and were then mixed with Hoechst in equal proportions, and then the mixture was incubated at room temperature for 10 minutes to stain the nuclei. The number of the nuclei was counted, and then the amount of phospholipid contained in the nuclei was measured in the same manner as in Section 2-2-4 to make the suspension have the same lipid concentration as the liposome suspension. The liposome suspension and the suspension of the isolated nuclei were mixed in a ratio of liposome:nuclei= 9:2, and then the mixture was incubated at 37°C for 1 hour and then centrifuged at 1400g at 4°C for 1 minute. The pellet was washed with Import buffer twice, and then suspended in an appropriated amount of Import buffer. An appropriate amount of the suspension was dropped onto a glass slide, covered with a cover glass, and the cover glass was fixed to the glass slide with nail polish. The thus prepared glass slide was observed by CLSM.

(2) Observation result

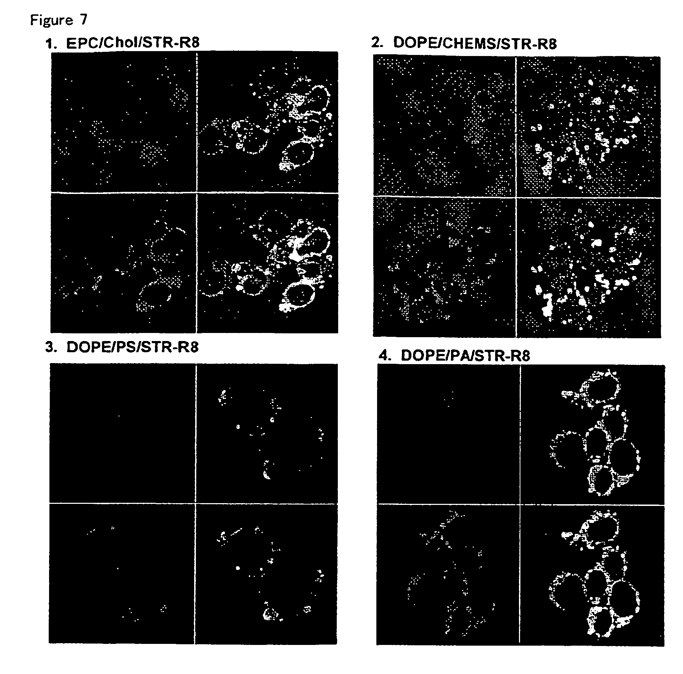

The observation results are shown in Fig. 7. In the case of the liposome having a lipid composition of EPC/Chol/STR-R8, membrane fusion did not occur, and therefore red dots resulting from the liposomes and green dots resulting from GFP entrapped in the liposomes were colocalized around nuclear membranes. On the other hand, in the case of the other three kinds of membrane fusible liposomes, many red portions resulting from the liposomes were present alone, and green portions resulting from GFP were distributed alone along the outline of the nuclei. This suggest that the liposomes having lipid compositions of DOPE/CHEMS, DOPE/PA, and DOPE/PS were fused with nuclear membranes so that part of GFP entrapped in the liposomes was distributed in the gap between two membranes of the nuclei. Further, in the case of the liposome having a lipid composition of DOPE/CHEMS, green portions resulting from GFP and blue portions resulting from the nuclei were colocalized so that some light blue portions were observed. This suggests that some of the liposomes were fused with nuclei and GFP was transferred into the nuclei.

3-4. Summary

It has been found that by modifying lipids with STR-R8, some lipids can have an improved ability to bind to nuclear membranes without significantly changing their ability to fuse with nuclear membranes, and some lipids can have not only an improved ability to bind to nuclear membranes but also an improved ability to fuse with nuclear membranes. Contrary to expectations, it has been found that neutral lipids can also have an improved ability to bind to and fuse with nuclear membranes by modifying them with STR-R8. Further, visualization of membrane fusion was achieved by a confocal microscope image, thereby significantly increasing the possibility of gene delivery by membrane fusion with nuclear membranes.

(Example 2)

Screening of lipids having ability to fuse with nuclear membranes

1. Screening using FRET

1-1. Preparation of membrane-labeled liposomes

(1) DOPE:lipid= 9:2

1 mM DOPE and a 1 mM lipid stock were mixed in a mole ratio of 9:2, and a $CHCl_3$ solution containing NBD-DOPE in an amount of 1 mol% of the total lipid concentration and a $CHCl_3$ solution containing Rho-DOPE in an amount of 0.5 mol% of the total lipid concentration were added thereto, and $CHCl_3$ was further added thereto. The thus obtained mixture was dried in an atmosphere of nitrogen gas to prepare lipid film. Then, 10 mM HEPES buffer as an internal aqueous phase was added to the lipid film so that the total lipid concentration became 0.55 mM to hydrate the lipid film. Then, ultrasonic treatment was carried out to prepare liposomes.

(2) DOPE:lipid= 5:5

1 mM DOPE and a 1 mM lipid stock were mixed in a mole ratio of 5:5, and a $CHCl_3$ solution containing NBD-DOPE in an amount of 1 mol% of the total lipid concentration and a $CHCl_3$ solution containing Rho-DOPE in an amount of 0.5 mol% of the total lipid concentration were added thereto, and $CHCl_3$ was further added thereto. The thus obtained mixture was dried in an atmosphere of nitrogen gas to prepare lipid film. Then, 10 mM HEPES buffer as an internal aqueous phase was added to the lipid film so that the total lipid concentration became 0.55 mM to hydrate the lipid film. Then, ultrasonic treatment was carried out to prepare liposomes.

1-2. Evaluation of ability of liposomes to fuse with nuclear membranes using FRET

Nuclei were isolated from Hela cells, and the amount of phospholipid contained in the nuclei was measured to determine the amount of PC contained in the nuclei. It has been reported that the amount of PC contained in the nuclear membrane is 55%, and therefore the total lipid concentration of the nuclei was determined on the assumption that the amount of PC contained in the nuclear membrane was 55%, and the suspension of the isolated nuclei was diluted so that the total lipid concentration was 0.13 mM (that is a quarter of Example 1).
A Probe Lip prepared by the method described in Section 1-1 and the suspension of the isolated nuclei were mixed in a ratio of Probe Lip: nuclei= 1: 9 in a 1.5 mL sample tube, and the thus obtained mixture was incubated at 37°C for 30 minutes. A control was prepared by mixing the Probe Lip and Import buffer in a ratio of 1:9 and incubating the mixture at 37°C for 30 minutes. A control for measuring a maximum fluorescence intensity was prepared by mixing the Probe Lip and 0.5% Triton in a ratio of 1:9 and incubating the mixture at 37°C for 30 minutes. After the completion of incubation, the fluorescence intensity of each of the samples (excitation wavelength: 470 nm, absorption wavelength: 530 nm, 590 nm) was measured to obtain a spectrum. Based on the measured fluorescence intensity at 530 nm, TF was calculated in the same manner as in Example 1.

1-1-3. Result

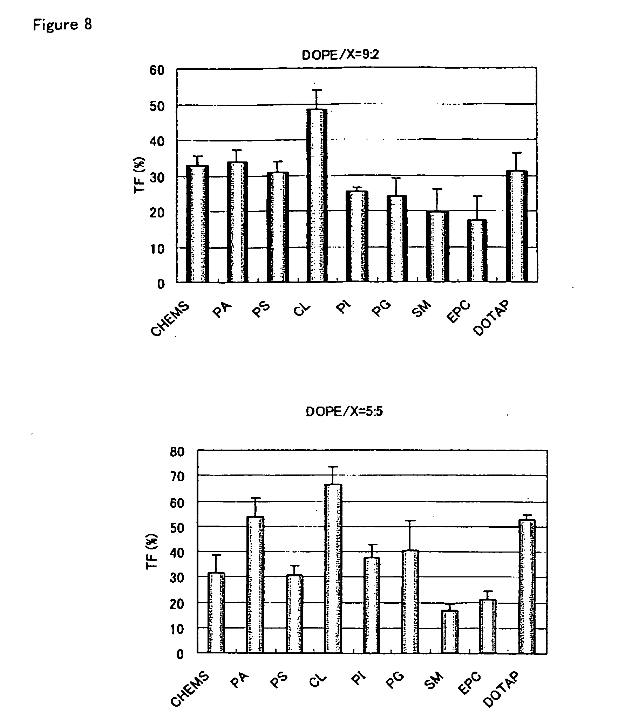

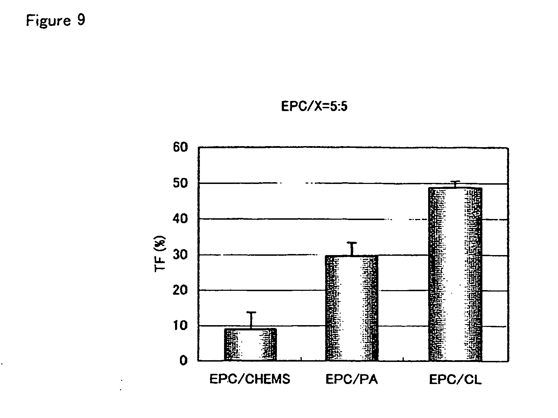

Various liposomes were prepared using the following lipids by the method described in Section 1-1, and an experiment was carried out using the liposomes. The results (TF) are shown in Fig. 8.
EPC($\pm$), SM($\pm$), DOTAP(+), PA(-), PS(-), PI(-), Cardiolipin(-), PG(-), CHEMS(-)
As shown in Fig. 8, liposomes prepared by mixing DOPE and a lipid in a ratio of 5:5 have a higher ability to fuse with nuclear membranes than liposomes prepared by mixing DOPE and a lipid in a ratio of 9:2. This result indicates that liposomes having a lower DOPE (membrane-fusible lipid) content have a higher ability to fuse with nuclear membranes. Particularly, in the case of a liposome using PA and a liposome using CL, their ability to fuse with nuclear membranes was significantly improved. Also in the case of a liposome using DOTAP as a positively-charged lipid, its ability to fuse with nuclear membrane was improved. However, in this case, it can be considered that positive charge was increased by increasing the ratio of DOTAP and therefore the affinity of the liposome using DOTAP for negatively-charged nuclear membranes was increased. Since PA, CL, and CHEMS showed a high ability to fuse with nuclear membranes, the DOPE (membrane-fusible lipid)-independent ability of PA, CL, and CHEMS to fuse with nuclear membranes was examined by preparing liposomes using EPC instead of DOPE. The results are shown in Fig 9. As shown in Fig. 9, in the case of a liposome using EPC/CHEMS, membrane fusion hardly occurred. On the other hand, a liposome using PA and a liposome using CL still had a high ability to fuse with nuclear membranes even when DOPE was replaced with EPC. As a result, it has been found that the negatively-charged lipids have an affinity for nuclear membranes. In addition, it has also be found that PA and CL are lipids having a particularly high affinity for nuclear membranes.

2. Visualization of lipids having ability to fuse with nuclear membranes by confocal laser microscope

2-1. Preparation of membrane-labeled liposomes entrapping GFP

A 1 mM lipid stock and a 1 mM DOPE stock were mixed in a specified mole ratio, and a CHCl$_3$ solution containing Rho-DOPE in an amount of 0.5 mol% of the total lipid concentration was added thereto, and then CHCl$_3$ was further added thereto. The thus obtained mixture was dried in an atmosphere of nitrogen gas to prepare lipid film. Then, GFP was diluted with 10 mM HEPES to 0.125 mg/mL, and the HEPES solution of GFP was added as an internal aqueous phase to the lipid film so that the total lipid concentration became 0.55 mM to hydrate the lipid membrane. Then, ultrasonic treatment was carried out to prepare liposomes.

2-2. Analysis by confocal laser microscope

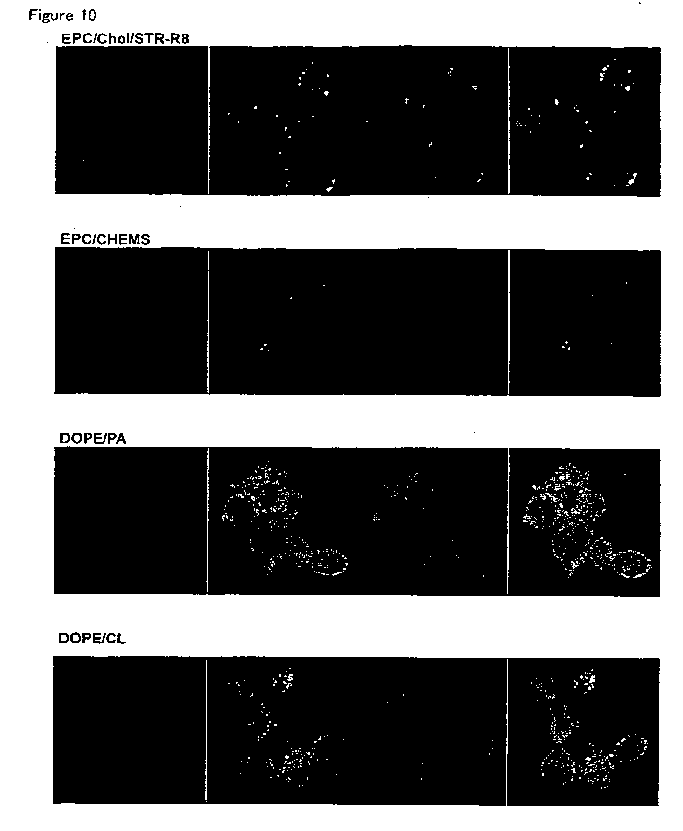

A liposome was prepared by mixing DOPE and PA (membrane-fusible lipids) in a ratio of 5:5, and a liposome was prepared by mixing DOPE and CL (membrane-fusible lipids) in a ratio of 5:5. Further, as controls, a liposome was prepared by mixing EPC, Chol, and STR-R8 in a ratio of 9:2:0.55 (bindability: O, fusibility: ×), and a liposome was prepared by mixing EPC and CHEMS in a ratio of 5:5 (bindability:×, fusibility:×). It is to be noted that all the liposomes were prepared by the method described in Section 2-1. Further, isolated nuclei were mixed with Hoechst in equal proportions, and the mixture was incubated at room temperature for 10 minutes to stain the nuclei. The amount of phospholipid contained in the nuclei was measured to make the suspension have the same total lipid concentration as the liposome suspension. The liposome suspension, and the suspension of the isolated nuclei were mixed in a ratio of liposomes: nuclei= 9:2, and the mixture was incubated at 37°C for 1 hour and then centrifuged at 1400g at 4°C for 1 minute. The pellet was washed with Import buffer twice, and was then suspended in an appropriate amount of Import buffer. An appropriate amount of the suspension was dropped onto a glass slide and was covered with a cover glass, and then the cover glass was fixed to the glass slide using nail polish. The thus prepared glass slide was observed with a confocal laser microscope. The results are shown in Fig. 10.
As shown in Fig. 10, in the case of the liposome using EPC/CHEMS not having an affinity for nuclear membranes at all, fluorescence was hardly observed around the nuclei. However, in the case of the liposome using EPC/Chol/STR-R8 having only a high ability to bind to nuclei, red dots resulting from the lipid and green dots resulting from GFP entrapped in the liposomes were colocalized so that some yellow dots were observed around the nuclei. On the other hand, in the case of the liposome using DOPE/PA and the liposome using DOEP/CL, many red portions resulting from the liposomes were present alone around the nuclei and green portions resulting from GFP were distributed along the outline of the nuclei. In addition, in the case of the liposome using DOPE/CL, the obtained confocal microscope image suggests that part of GFP entrapped in the liposomes localizes in the nuclei. This suggests that the liposome using DOPE/PA and the liposome using DOPE/CL fuse with nuclear membranes and then part of GFP entrapped in the liposomes can be delivered into the nuclei. This suggestion convinces us that these liposomes can permeate nuclear membranes by membrane fusion. Therefore, it can be considered that these liposomes can greatly contribute to future gene delivery.

(Example 3)

Screening of lipids to find endosomal escape-promoting lipids

In a case where a liposome containing two kinds of fluorescent pigments (NBD and Rhodamine) in its lipid membrane is present alone and the fluorescence spectrum of one of the fluorescent pigments and the excitation spectrum of the other fluorescent pigment are overlapped, the fluorescence of NBD is not usually observed even when NBD is irradiated with light having the excitation wavelength of NBD (this is because when NBD and Rhodamine coexist adjacent to each other, the fluorescence energy of NBD is used to excite Rhodamine; and this phenomenon is called "FRET" (Fluorescence Resonance Energy Transfer). When such a liposome is fused with another lipid membrane (e.g., cell membrane), the liposomal lipid is diluted with the lipid membrane as a receiver so that the distance between NBD and Rhodamine contained in the liposomal membrane is increased, and therefore a phenomenon called FRET is cancelled. Utilizing such a phenomenon, screening of lipids was performed to find lipids (endosomal escape-promoting lipids) having high ability to fuse with cell membranes (endosomal membranes). It is to be noted that in this research, a pH at which liposomes and cells are brought into contact with each other is adjusted to 6.5 that is the initial pH inside the endosome to reproduce endosomal environments, and membrane fusion between liposomes and endosomal membranes is quantitatively evaluated by utilizing FRET.

1. Method

(1) Preparation of liposomes

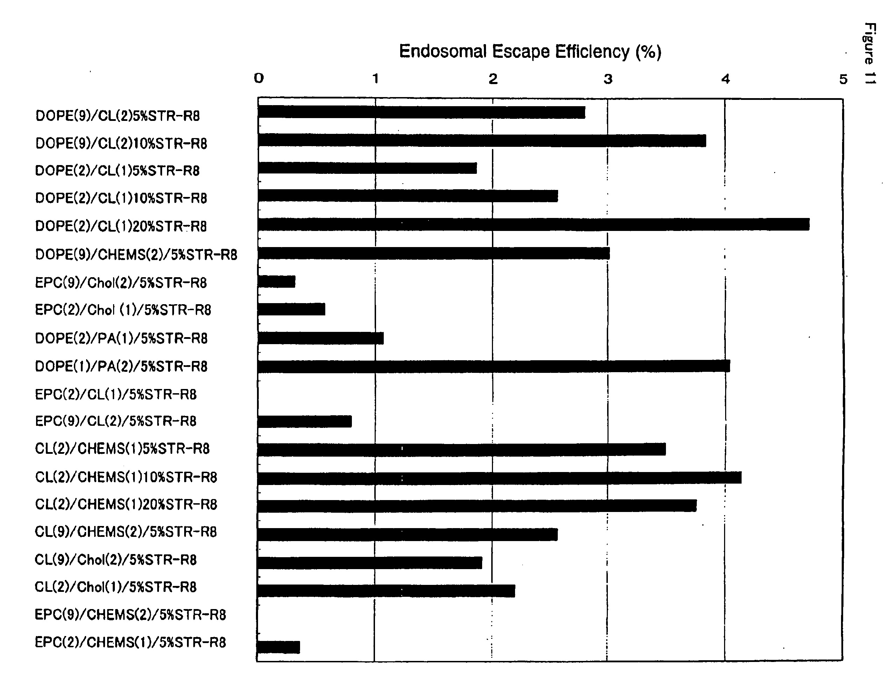

Lipid film doubly labeled with NBD-DOPE and Rhodamine-DOPE was prepared in a test tube, and HEPES-Glucose (5%) was added to the lipid film to sufficiently hydrate the lipid film. Then, the hydrated lipid film was ultrasonically treated with a bath-type sonicator to prepare liposomes. If necessary, STR-R8 was added (in an amount of 5, 10, or 20 mol% of the amount of lipids). A final liposome concentration was 0.55 mM, the amount of NBD-DOPE was 1% of the total amount of lipids, and the amount of Rhodamine-DOPE was 0.5% of the total amount of lipids. After the preparation of the liposomes, the particle size and Z-potential thereof were measured. It is to be noted that the lipid compositions of various liposomes are shown in Fig. 11 wherein values within parentheses represent mole ratios.

(2) Preparation of sample and measurement of fluorescence intensity

Before 24 hours from the experiment, $5 \times 10^5$ NIH3T3 cells per well were plated in a 6-well plate, and were incubated for 24 hours. After the completion of 24h-incubation, DMEM was removed and then the cells were washed with DMEM (Serum Free). Then, 800 $\mu$L of DMEM (pH 6.5, Serum Free) and 200 $\mu$L of liposomes were added per well to incubate the cells for 1 hour (Cell suspension:Liposome= 4:1). After 1 hour, the solvent and liposome solution not fused with cell membranes were removed, and then the cells were washed with PBS. The cells were collected in trypsin, and the cell suspension was centrifuged at 10,000 rpm at 4°C for 5 minutes, and then the supernatant was removed. The remaining cells were suspended in 100 $\mu$L of HEPES-(5%)Glucose, and the thus obtained cell suspension was divided into two. To one of the cell suspensions, 50 $\mu$L of HEPES-(5%)Glucose was further added, and the fluorescence intensity of the cell suspension was defined as F'. To the other cell suspension, 50 $\mu$L of Triton was added to lyse the cells (final Triton concentration: 0.5%), and the fluorescence intensity of the cell suspension was defined as F'max. The fluorescence intensity of a blank (Buffer:Liposome= 80 uL:20 $\mu$L) was defined as F0, and the fluorescence intensity of a control obtained by breaking liposomes with Triton (final concentration: 0.5%) (0.5% Triton:Liposome= 80 $\mu$L:20 $\mu$L) was defined as Fmax. The amount of protein contained in each of the samples for measuring the fluorescence intensities F' and F'max was measured using a BCA TM Protein Assay Kit (PIERCE), and then the amount of protein contained in each of the sample tubes and the amount of protein contained in one well of the 6-well plate were calculated from the absorbances of the samples for measuring F' and F'max.

(3) Analysis of fluorescence intensity

The fluorescence intensities of the samples were measured at 530 nm that is the fluorescence wavelength of NBD. By substituting the measured fluorescence intensity data of F', F0, F'max, and Fmax into the following formulas to determine Transfer Efficiency (TF) and Binding Efficiency (BF). The amount of protein contained in the sample for measuring F' was defined as P(F'), the amount of protein contained in the sample for measuring F'max was defined as P(F'max), and the amount of protein contained in one well was defined as P(total). The fluorescence intensity of a sample containing all the cells collected was determined by calculation and was defined as F' collected (a), but the liposome concentration of the sample was condensed 10-fold as compared to the concentration of liposomes applied when the cells had been collected, and therefore F' collected was decreased by a factor of ten for correction and the corrected F' collected was defined as F' recovered (b). The fluorescence intensity of a sample obtained by breaking liposomes bound to all the cells with 0.5% Triton was determined by calculation and defined as F'max collected (c), but the liposome concentration of the sample was condensed 10-fold as compared to the concentration of liposomes applied when the cells had been collected, and therefore the F'max collected was decreased by a factor of ten for correction and the corrected F'max was defined as F'max recovered (d). Then, BE(e) and TF ((f), (g)) were calculated using the following formulas, and the efficiency of delivering the vector into the cytoplasm by membrane fusion was calculated from BE and TF and was defined as endosomal escape efficiency (h).

$$\text{F' collected} = \text{F'/P(F')} \times \text{P(total)} \cdots \text{(a)}$$

$$\text{F' recovered} = \text{F' collected/10} \cdots \text{(b)}$$

$$F'\ max\ collected\ =\ F'\ max/P(F'\ max)\ \times\ P(total)\ \cdots\ (c)$$

$$F'\ max\ recovered\ =\ F'\ max\ collected/10\ \cdots\ (d)$$

$$BE\ =\ (F'\ max\ recovered/F\ max)\ \times\ 100\ \cdots\ (e)$$

$$F\ =\ F'\ recovered\ \times\ (1/BE)$$

$$=\ F'\ recovered\ \times\ (F\ max/F'\ max\ recovered)\ \cdots\ (f)$$

$$TF\ =\ [(F-F0)/(F\ max\ -\ F0)]\ \times\ 100\ \cdots\ (g)$$

$$Endosomal\ Escape\ Efficiency\ =\ BE\ (Binding\ Efficiency)\ \times$$

$$TF\ (Transfer\ Efficiency)\ \times\ 100\ (\%)\ \cdots\ (h)$$

(4). Transfection Assay

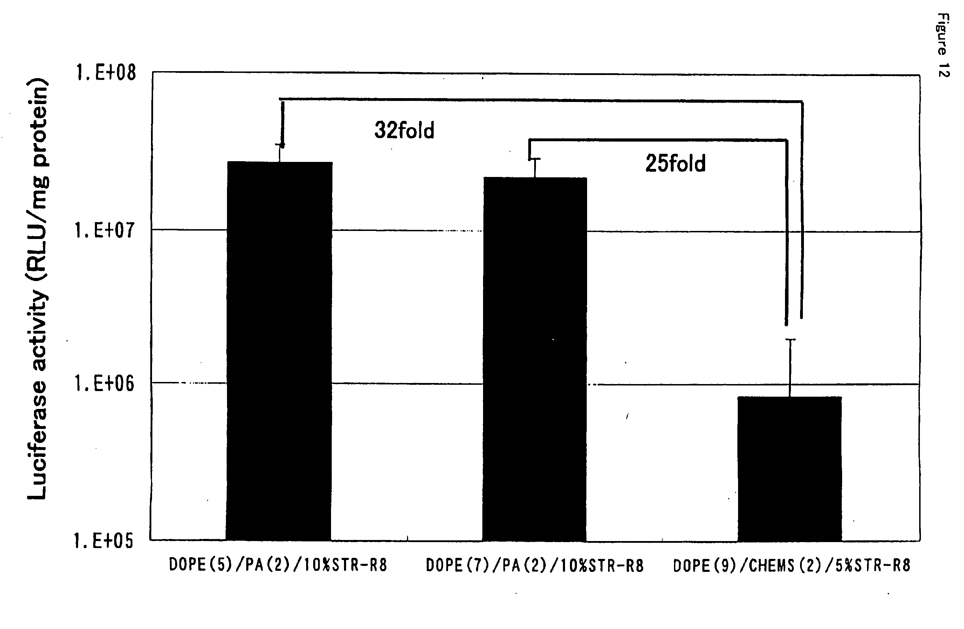

pDNA was condensed with Poly-L-Lysine to prepare liposomes entrapping condensed DNA (final lipid concentration: 0.55 mM). The surface of the liposomes was modified with an appropriate amount of STR-R8. The lipid compositions of various liposomes are shown in Fig. 12 wherein values within parentheses represent mole ratios. As the pDNA, a luciferase gene expression plasmid, pcDNA3.1(+)luc was used. The pcDNA3.1(+)luc is a plasmid DNA having a total length of about 7 kbp and containing a CMV promoter and a luciferase gene connected downstream of the CMV promoter. Such a plasmid DNA was prepared by introducing a luciferase gene cut out from a pGL3 plasmid (manufactured by Promega) using a restriction enzyme into a plasmid pcDNA3.1(+) (manufactured by Invitrogen). 4 $\times 10^4$ NIH3T3 cells were plated per well in a 24 well-plate, and were then incubated at 37°C for 24 hours in a 5% $CO_2$ incubator. After 24 hours, the cells were washed with 500 $\mu$L of DMEM(- serum), and then the liposomes were added so that the amount of DNA contained in 250 $\mu$L of DMEM (- serum) was 0.04 $\mu$g. Then, the cells were further incubated at 37°C for 3 hours in a 5% $CO_2$ incubator. After 3 hours, 1 mL of DMEM (+ serum) was added, and then the cells were further incubated at 37°C for 21 hours in a 5% $CO_2$ incubator. After 21 hours, the cells were washed with 500 $\mu$Lof PBS 1X, and then 75 $\mu$L of Lysis Reporter Buffer 1X was added to the cells, and the cell suspension was frozen at -80°C for 30 minutes. The cell suspension was melt at 4°C and was centrifuged at 15,000 rpm at 4°C for 4 minutes. Then, 50 $\mu$Lof the supernatant was transferred into another tube. 20 $\mu$L of the sample and 50 $\mu$L of a luciferase substance were mixed to measure the luciferase activity of the sample. Further, 5 $\mu$L of the remaining sample was diluted with 20 $\mu$L of $H_2O$ to measure the amount of protein. The measured luciferase activity was divided by the amount of protein to determine luciferase activity per unit amount of cellular protein (R.L.U/mg protein).

2. Result and consideration

(1) Screening of lipids to find endosomal membrane fusion-promoting lipids based on endosomal escape efficiency

Endosomal escape efficiency was compared between various liposomes constituted by different lipids and containing two kinds of fluorescent pigments and STR-R8 (for the purpose of selecting a macropinocytosis pathway) (Fig. 11). DOPE/CHEMS/STR-R8 conventionally known as a lipid showing high ability to fuse with membranes in endosomal environments was defined as a positive control. A liposome composition showing an endosomal escape efficiency higher than that of DOPE/CHEMS/STR-R8 (3%) was defined as an endosomal membrane fusion-promoting lipid. As a result, it has been found that lipids (phosphatidic acid (PA) and cardiolipin (CL)) have high ability to fuse with membranes (see Fig. 11). This result could not be predicted from conventional common knowledge about their chemical structure. From the result, it has been judged that CL and PA are lipids having higher ability to fuse with endosomal membranes than CHEMS. Since these lipids have negative charge and cell membranes and endosomal membranes are also rich in negatively-charged lipids, it has been predicted that CL or PA and cell membranes/endosomal membranes would repel each other. On the contrary, CL and PA had higher ability to fuse with membranes than positively-charged lipids. This is a world's first discovery. In order to examine whether or not these lipids can actually promote the intracellular trafficking of carriers, transfection assay was performed using a liposome having a lipid membrane containing PA. As a result, the luciferase activity of a liposome having a conventional lipid composition (DOPE/CHEMS) was about $10^6$, but the luciferase activity of a liposome containing PA was 30-fold or more higher than that of the liposome having a conventional lipid composition (see Fig. 12). It is to be noted that the amount of STR-R8 of the liposome using DOPE/CHEMS (5%) was lower than that of the liposome using DOPE/PA (10%), but the luciferase activity of the liposome using DOPE/CHEMS was not changed even by increasing the amount of STR-R8 to 10%. This indicates that luciferase activity is not increased by increasing the amount of STR-R8. These results suggest that by using the endosomal escape-promoting lipids found in this research, it is possible to significantly improve gene expression using a nonviral gene delivery system. Heretofore, various researches have been made from the viewpoint of the process of endosomal escape, but researches to find a lipid having a high ability to fuse with endosomal membranes based on quantitative evaluation of membrane fusion as such have never been performed. Therefore, the results of this research are world's first findings. It can be expected that the findings of this research will significantly improve gene delivery technology in vivo using nonviral gene vectors. Particularly, endosomal escape is a bottleneck for siRAN delivery recently receiving attention as "feasible gene therapy", and therefore it can be expected that unprecedentedly high therapeutic effect will be obtained by using the novel lipids found in this research. Therefore, it can be considered that this research is a fundamental technology essential in the fields of gene therapy such as siRNA delivery and immunotherapy using DNA vaccines.

(Example 4) Analysis of system for delivering entrapped substance by membrane fusion

(1) Preparation of membrane-labeled MLV entrapping GFP

10 mM DOPE and a 10 mM lipid stock were mixed in a mole ratio of 5:5 in a glass test tube, and then a $CHCl_3$ solution containing Rho-DOPE in an amount of 1 mol% of the total lipid concentration was added thereto, and then $CHCl_3$ was further added thereto. Then, the thus obtained mixture was dried in an atmosphere of nitrogen gas to prepare lipid film. Then, GFP was diluted with 10 mM HEPES Buffer to 0.125 mg/mL, and the HEPES solution of GFP was added as an internal aqueous phase so that the total lipid concentration became 0.55 mM to hydrate the lipid membrane. Then, ultrasonic treatment was carried out to prepare MLVs.

(2) Preparation of membrane-labeled SUV entrapping GFP

10 mM DOPE and a 10 mM lipid stock were mixed in a mole ratio of 5:5 in a glass test tube, and then a $CHCl_3$ solution containing Rho-DOPE in an amount of 1 mol% of the total lipid concentration was added thereto, and then $CHCl_3$ was further added thereto. Then, the thus obtained mixture was dried in an atmosphere of nitrogen gas to prepare lipid film. Then, GFP was diluted with 10 mM HEPES Buffer to 0.125 mg/mL, and the HEPES solution of GFP was added as an internal aqueous phase to the lipid film so that the total lipid concentration became 0.55 mM to hydrate the lipid membrane for 10 minutes. Then, ultrasonic treatment was carried out using a probe-type sonicator for 10 minutes to prepare SUVs.

(3) Analysis using confocal laser microscope

MLV and SUV were prepared by the method described in (1) and the method described in (2), respectively, using DOPE/PA (5:5) as a fusible lipid. Further, MLV and SUV were prepared by the method described in (1) and the method described in (2), respectively, using DOPE/CL (5:5) as a fusible lipid. Then, isolated nuclei were mixed Hoechst in equal proportions, and then the mixture was incubated at room temperature for 10 minutes to stain the nuclei. Then, the liposomes and the nuclei were mixed in a ratio of 9: 2 (w/w), and the mixture was incubated at 37°C for 1 hour, and was then centrifuged at 1400xg at 4°C for 30 seconds. The thus obtained pellet was washed with Import buffer twice, and was then suspended in an appropriate amount of Import Buffer. An appropriate amount of the thus obtained suspension was dropped onto a glass slide and covered with a cover glass, and the cover glass was fixed to the glass slide using nail polish. The thus prepared glass slide was observed by a confocal laser microscope.

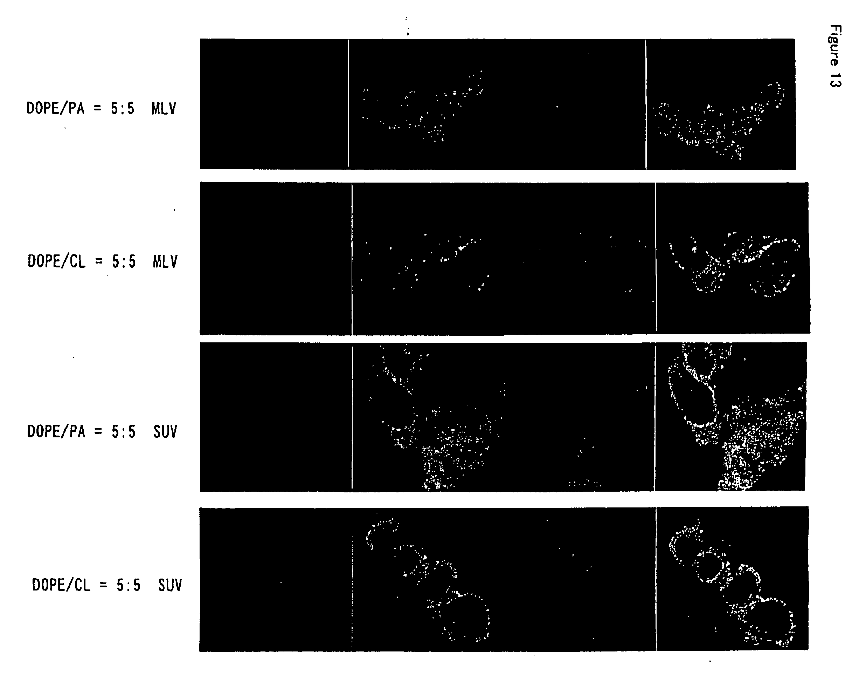

The observation results are shown in Fig. 13. It is to be noted that in Fig. 13, a panel showing the localization of nuclei stained with Hoechst (blue), a panel showing the localization of GFP (green), a panel showing the localization of Rhodamine-labeled lipid (red), and a panel obtained by overlapping these three panels are arranged from the left to the right.

MLV is a vector having a multilamellar structure. In either of the case using the MLV containing PA and the case using the MLV containing CL, the lipid (red) was localized around the nuclei, but the GFP (green) entrapped in the MLVs was dispersed inside the nuclei. On the other hand, in the case of the SUVs having a unilamellar structure, the lipid (red) was localized on the surface of the nuclear membranes and the GFP (green) entrapped in the SUVs was localized alone along the outline of the nuclei. This result suggests that the SUVs having only one membrane could not permeate the bilamellar structure of the nuclei and were therefore trapped in the gap between two nuclear membranes, whereas the MLVs permeated the bilamellar structure of the nuclei stepwise by membrane fusion and then the GFP entrapped in the MLVs was released into the nuclei.

(Example 5) Evaluation of gene expression in nondividing cells

(1) Preparation of DNA core

Plasmid DNA (pEGFP/Luc) and Protamine were diluted with HEPES Buffer, and then the plasmid DNA (0.1 mg/mL) was dropped into the Protamine (0.1 mg/mL) under the vortex condition (weight ratio= 1:1.5, N/P ratio= 2:2) to prepare a DNA core.

(2) Preparation of liposomes

1 mM DOPE and a 1 mM lipid stock were mixed in a predetermined mole ratio in a glass test tube, and then a $CHCl_3$ solution was further added thereto, and then the thus obtained mixture was dried in an atmosphere of nitrogen gas to prepare lipid film. The pDNA/Protamine core prepared by the method described in the above (1) was added as an internal aqueous phase to the lipid film so that the total lipid concentration became 0.55 mM to hydrate the lipid film. Then, ultrasonic treatment was carried out to prepare liposomes. Then, STR-R8 (2 mg/mL) was added to allow the liposomes to be positively charged, and the liposomes and the STR-R8 were mixed with a pipette, and then the mixture was incubated at room temperature for 30 minutes.
In this way, a liposome A containing DOPE/CHEMS (9 : 2) and STR-R8 (5%), a liposome B containing DOPE/CHEMS (5 : 5) and STR-R8 (5%) , a liposome C containing EPC/CHEMS (9 : 2) and STR-R8 (5%), a liposome D containing DOPE/PA (5:5) and STR-R8 (20%), a liposome E containing DOPE/CL (5:5) and STR-R8 (20%), and a liposome F containing DOPE/PA (7:2) and STR-R8 (10%) were prepared. It is to be noted that these liposomes A to F are sometimes generically called "MENDs". MENDs are liposomes having one or two or more lipid membranes.

(3) Preparation of liposome-liposomes

(a) Inner lipid membranes containing DOPE/PA (5:5) and outer lipid membranes containing DOPE/PA (7:2)

10 mM DOPE and 10mM PA were mixed in a mole ratio of 5:5 in a glass test tube, and then $CHCl_3$ was added thereto. Then, the thus obtained mixture was dried in an atmosphere of nitrogen gas to prepare lipid film. Then, 10 mM HEPES buffer was added as an internal aqueous phase to the lipid film so that the total lipid concentration became 0.55 mM to hydrate the lipid film for 10 minutes. Then, ultrasonic treatment was carried out using a probe type-sonicator for 10 minutes to prepare SUVs. Then, the SUPS containing DOPE/PA (5 : 5) and the pDNA/protamine core prepared by the method described in the above (1) were mixed in a ratio of 2:1 to prepare bilamellar liposomes. To the bilamellar liposome solution, STR-R8 (2 mg/mL) was added in an amount of 20% of the total lipid concentration, and then the mixture was incubated at room temperature for 30 minutes.
Then, SUVs containing DOPE/PA (7:2) and the bilamellar liposomes were mixed in a ratio of 2:1 to prepare tetralamellar liposomes. To the tetralamellar liposome solution, STR-R8 (2 mg/mL) was added in an amount of 10% of the total lipid concentration, and the mixture was incubated at room temperature for 30 minutes.
In this way, a tetralamellar liposome A having two inner lipid membranes containing DOPE/PA (5:5) and two outer lipid membranes containing DOPE/PA (7:2) was prepared.

(b) Inner lipid membranes containing DOPE/CL (5:5) and outer lipid membranes containing DOPE/PA (7:2)

10 mM DOPE and 10mM CL were mixed in a mole ratio of 5:5 in a glass test tube, and then $CHCl_3$ was added thereto. Then, the thus obtained mixture was dried in an atmosphere of nitrogen gas to prepare lipid film. Then, 10 mM HEPES buffer was added as an internal aqueous phase to the lipid film so that the total lipid concentration became 0.55 mM to hydrate the lipid film for 10 minutes. Then, ultrasonic treatment was carried out using a probe type-sonicator for 10 minutes to prepare SUVs.

Then, the SUVs containing DOPE/PA (5:5) and the pDNA/protamine core prepared by the method described in the above (1) were mixed in a ratio of 2:1 to prepare bilamellar liposomes. To the bilamellar liposome solution, STR-R8 (2 mg/mL) was added in an amount of 20% of the total lipid concentration, and then the mixture was incubated at room temperature for 30 minutes.

Then, SUVs containing DOPE/PA (7:2) and the bilamellar liposomes were mixed in a ratio of 2:1 to prepare tetralamellar liposomes. To the tetralamellar liposome solution, STR-R8 (2 mg/mL) was added in an amount of 10% of the total lipid concentration, and the mixture was incubated at room temperature for 30 minutes.

In this way, a tetralamellar liposome B having two inner lipid membranes containing DOPE/CL (5:5) and two outer lipid membranes containing DOPE/PA (7:2) was prepared.

(c) Inner lipid membranes containing DOPE/PA (5:5) (DOPE/CL= 5:5) and outer lipid membranes containing EPC/ CHEMS (9:2)

SUVs containing DOPE/PA (5:5) (DOPE/CL = 5:5) were prepared by the method described above. Then, 10 mM EPC and 10mM PA (CL) were mixed in a mole ratio of 9:2 in a glass test tube, and then $CHCl_3$ was added thereto. Then, the thus obtained mixture was dried in an atmosphere of nitrogen gas to prepare lipid film. Then, 10 mM HEPES buffer was added as an internal aqueous phase to the lipid film so that the total lipid concentration became 0.55 mM to hydrate the lipid film for 10 minutes. Then, ultrasonic treatment was carried out using a probe type-sonicator for 10 minutes to prepare SUVs.

Then, the SUVs containing DOPE/PA (5:5) (DOPE/CL= 5:5) and the pDNA/protamine core prepared by the method described in the above (1) were mixed in a ratio of 2:1 to prepare bilamellar liposomes. To the bilamellar liposome solution, STR-R8 (2 mg/mL) was added in an amount of 20% of the total lipid concentration, and then the mixture was incubated at room temperature for 30 minutes.

Then, SUVs containing EPC/CHEMS (9:2) and the bilamellar liposomes were mixed in a ratio of 2:1, and an appropriate amount of 1N HCl was added thereto to induce membrane fusion to prepare tetralamellar liposomes. To the tetralamellar liposome solution, STR-R8 (2 mg/mL) was added in an amount of 5% of the total lipid concentration, and the mixture was incubated at room temperature for 30 minutes.

In this way, a tetralamellar liposome C having two inner lipid membranes containing DOPE/PA (5:5) and two outer lipid membranes containing EPC/CHEMS (9:2) and a tetralamellar liposome D having two inner lipid membranes containing DOPE/CL (5:5) and two outer lipid membranes containing EPC/CHEMS (9:2) were prepared.

(d) Inner lipid membranes containing EPC/CHEMS (9:2) and outer lipid membranes containing DOPE/PA (7:2) (EPC/ CHEMS= 9:2)

SUVs containing EPC/CHEMS (9:2) and SUVs containing DOPE/PA (7:2) were prepared by the method described above. Then, the SUVs containing EPC/CHEMS (9:2) and the pDNA/Protamine core prepared by the method described in the above (1) were mixed in a ratio of 2:1, and then an appropriate amount of 1N HCl was added thereto to induce membrane fusion to prepare bilamellar liposomes. To the bilamellar liposome solution, STR-R8 (2 mg/mL) was added in an amount of 5% of the total lipid concentration, and then the mixture was incubated at room temperature for 30 minutes.

Then, the SUVs containing DOPE/PA (7:2) or the SUVs containing EPC/CHEMS (9:2) and the bilamellar liposomes were mixed in a ratio of 2:1 to prepare tetralamellar liposomes. To the tetralamellar liposome solution, STR-R8 (2 mg/mL) was added in an amount of 5% of the total lipid concentration, and the mixture was incubated at room temperature for 30 minutes.

In this way, a tetralamellar liposome E having two inner lipid membranes containing EPC/CHEMS (9:2) and two outer lipid membranes containing DOPE/PA (7:2) and a tetralamellar liposome F having two inner lipid membranes containing EPC/CHEMS (9:2) and two outer lipid membranes containing EPC/CHEMS (9:2) were prepared. It is to be noted that these tetralamellar liposomes A to F are sometimes generically called "T-MENDs".

(4) Transfection

JAWSII cells ($8.0 \times 10^4$ cells) were placed in a 24 well-plate, and then were cultured for 2 days. Then, a sample corresponding to 0.4 μg of DNA was diluted with α-MEM not containing antibiotic and FCS so that the total volume of the sample was 250 μL, and the dilute sample was added to the cells. Naked DNA and HEPES Buffer were diluted in the same manner to prepared Nefgative controls, and these negative controls were added to the cells and the cells were cultured in a 5% $CO_2$ incubator at 37°C. After 3 hours from addition of the sample, 500 μL of α-MEM containing 10% FCS was added to the cells to again culture the cells in the 5% $CO_2$ incubator at 37°C. The cells were collected after 24 hours from addition of the sample, and luciferase activity and the amount of protein were measured. It is to be noted that in this experiment, JAWSII cells that are nondividing cells of immune system (mouse myeloid dendritic cells) were used for the purpose of reducing the influence of cell division on gene expression.

(5) Evaluation of gene expression

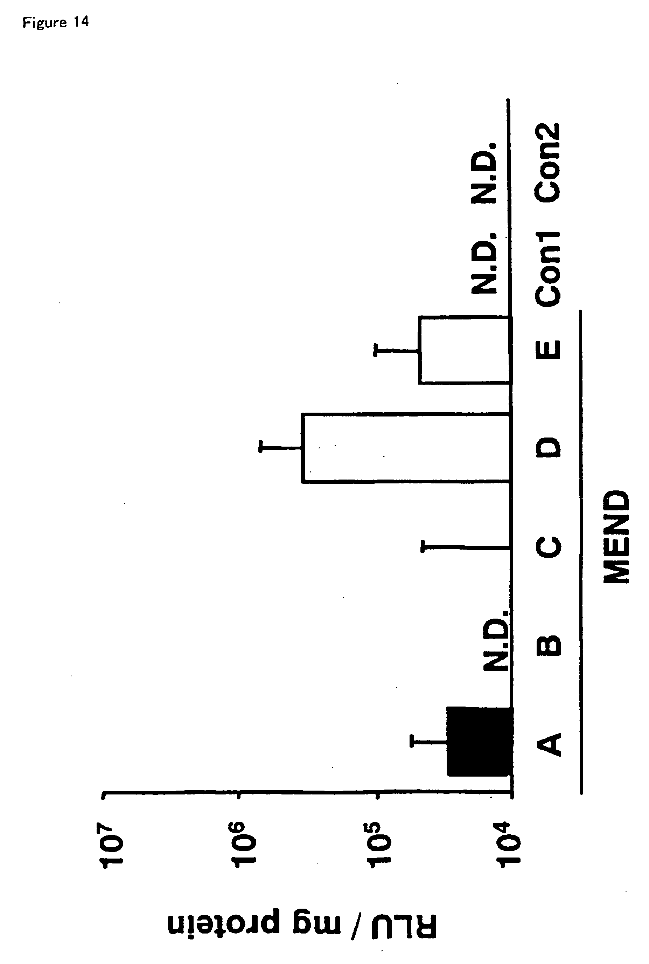

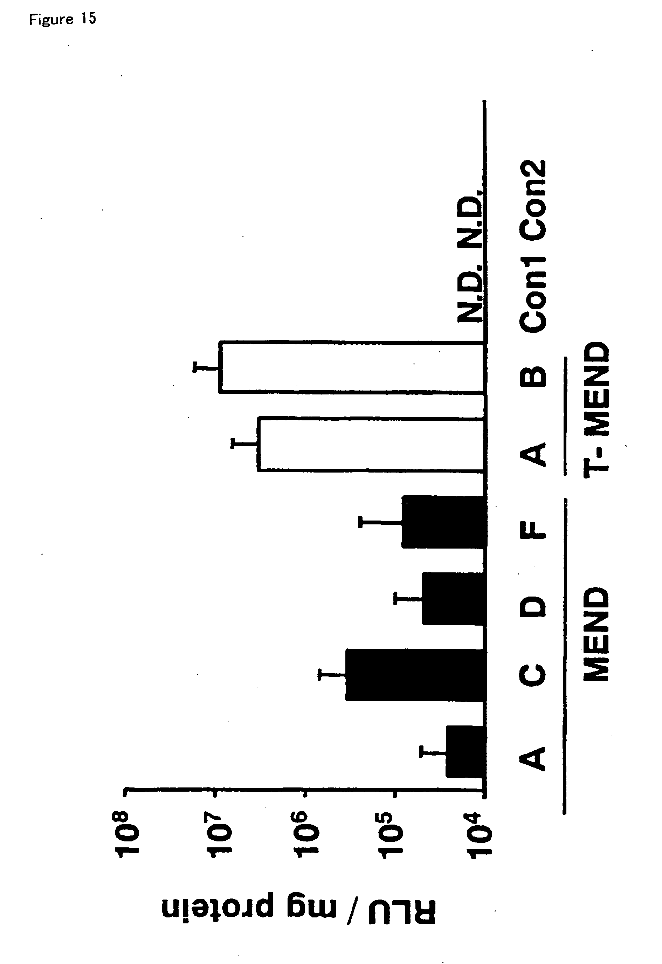

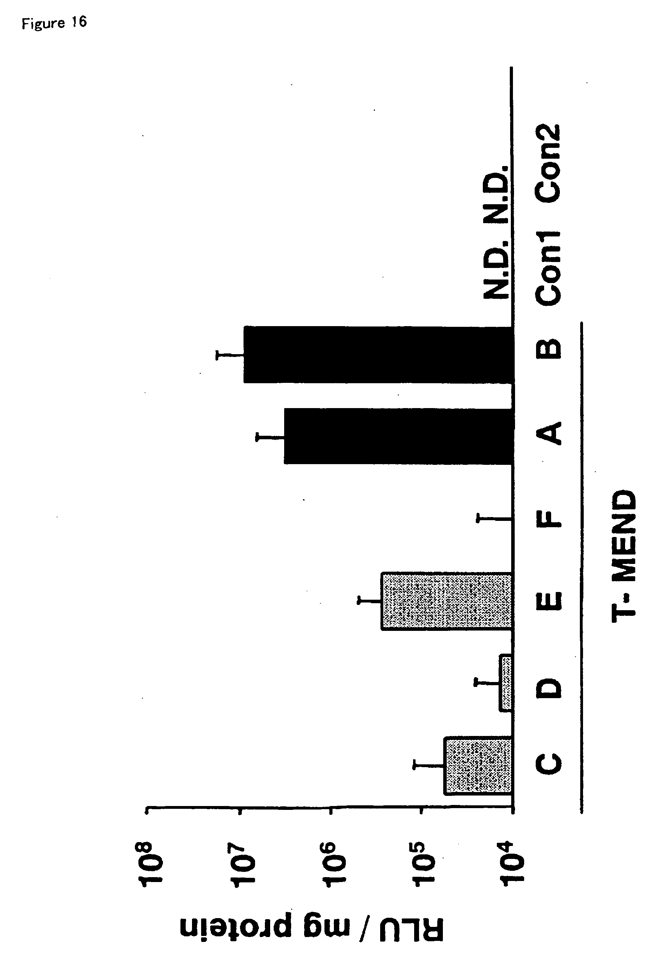

The measurement results of the efficiency of gene expression using the MENDs are shown in Fig. 14. It is to be noted that in Fig. 14, "Conl" represents a case where naked DNA was added and "Con2" represents a case where only cells were used. The same goes for Figs. 15 and 16.
As compared to the cases of DOPE/CHEMS (liposomes A and B) and EPC/CHEMS (liposome C), a significant increase in gene expression efficiency was shown in the cases of DOPE/PA (liposome D) and DOPE/CL (liposome E).
The measurement results of the efficiency of gene expression using the T-MENDs are shown in Figs. 15 and 16. In the case of using the T-MENDs, the efficiency of gene expression was higher than in the case of using the MENDs. A comparison was made between the tetralamellar liposomes A and B, the efficiency of gene expression was higher in the case of using CL having higher ability to fuse with nuclear membranes than in the case of using PA. This result suggests that the T-MENDs permeate membrane structures constituting a cell stepwise by membrane fusion. Therefore, it can be considered that the T-MENDs are vectors which can be used for non-dividing cells such as JAWSII cells.
The inner membranes and outer membranes of T-MENDs were changed to those containing EPC/CHEMS (9:2) having a low affinity for nuclear membranes or cell membranes (see fig. 16), and as a result, a significant decrease in the efficiency of gene expression was shown in every case. From the result, it has become apparent that when the outer membranes are changed to those containing EPC/CHEMS, the step of cellular entry becomes a rate-limiting step, and when the inner membranes are changed to those containing EPC/CHEMS, the step of nuclear entry becomes a rate-limiting step. It can be considered that this result strongly suggests that cellular entry and nuclear membrane permeation of the T-MENDs are carried out by membrane fusion.

**Claims**

1. A vector for delivering a target substance into a nucleus, comprising a lipid membrane structure having a first lipid membrane containing an anionic lipid and a second lipid membrane containing an anionic lipid being provided outside the first lipid membrane, wherein the anionic lipid of the first membrane is selected from a group consisting of cholesteryl hemisuccinate, phosphatidic acid and cardiolipin and the anionic lipid of the second membrane is selected from a group consisting of phosphatidic acid, cardiolipin, dialkyl phosphate and diacyl phosphate, and the first lipid membrane and the second lipid membrane contain a membrane permeable peptide.

2. The vector according to claim 1, wherein the amount of the anionic lipid contained in the first lipid membrane is 20 to 80% (mole ratio) of the total amount of lipids contained in the first lipid membrane.

3. The vector according to claims 1 or 2, wherein the anionic lipid contained in the first lipid membrane is phosphatidic acid or cardiolipin, and wherein the amount of the anionic lipid contained in the first lipid membrane is 40 to 60% (mole ratio) of the total amount of lipids contained in the first lipid membrane.

4. The vector according to any one of claims 1 to 3, wherein the first lipid membrane further contains dioleoylphosphatidyl ethanolamine.

5. The vector according to claim 4, wherein the amount of dioleoylphosphatidyl ethanolamine contained in the first lipid membrane is 20 to 80% (mole ratio) of the total amount of lipids contained in the first lipid membrane.

6. The vector according to any one of claims 1 to 5, wherein the amount of the anionic lipid contained in the second lipid membrane is 10 to 90% (mole ratio) of the total amount of lipids contained in the second lipid membrane.

7. The vector according to claim 6, wherein the second lipid membrane further contains dioleoylphosphatidyl ethanolamine.

8. The vector according to claim 7, wherein the amount of dioleoylphosphatidyl ethanolamine contained in the second lipid membrane is 10 to 90% (mole ratio) of the total amount of lipids contained in the second lipid membrane.

9. The vector according to any one of claims 1 to 8, wherein the membrane permeable peptide is a peptide having a protein transduction domain.

10. The vector according to claim 9, wherein the protein transduction domain is polyarginine.

11. The vector according to claim 10, wherein the polyarginine is composed of 4 to 20 consecutive arginine residues.

12. The vector according to any one of claims 9 to 11, wherein the membrane permeable peptide is present on the surface any one of the first lipid membrane and the second lipid membrane.

**Patentansprüche**

1. Vektor zur Abgabe einer Zielsubstanz in einen Zellkern, umfassend eine Lipidmembranstruktur, die eine erste Lipidmembran, die ein anionisches Lipid enthält, und eine zweite Lipidmembran, die ein anionisches Lipid enthält, die außerhalb der ersten Lipidmembran angeordnet ist, hat, wobei das anionische Lipid der ersten Membran aus der Gruppe, bestehend aus Cholesterylhemisuccinat, Phosphatidsäure und Cardiolipin, ausgewählt ist, und das anionische Lipid der zweiten Membran aus der Gruppe, bestehend aus Phosphatidsäure, Cardiolipin, Dialkylphosphat und Diacylphosphat, ausgewählt ist und die erste Lipidmembran und die zweite Lipidmembran ein Membranpermeables Peptid enthalten.

2. Vektor gemäß Anspruch 1, wobei die Menge des anionischen Lipids, die in der ersten Lipidmembran enthalten ist, 20 bis 80% (Molverhältnis) der Gesamtmenge an Lipiden, die in der ersten Lipidmembran enthalten ist, beträgt.

3. Vektor gemäß Anspruch 1 oder 2, wobei das anionische Lipid, das in der ersten Lipidmembran enthalten ist, Phosphatidsäure oder Cardiolipin ist, und wobei die Menge des anionischen Lipids, die in der ersten Lipidmembran enthalten ist, 40 bis 60% (Molverhältnis) der Gesamtmenge an Lipiden, die in der ersten Lipidmembran enthalten ist, ist.

4. Vektor gemäß einem der Ansprüche 1 bis 3, wobei die erste Lipidmembran außerdem Dioleoylphosphatidylethanolamin enthält.

5. Vektor gemäß Anspruch 4, wobei die Menge an Dioleoylphosphatidylethanolamin, die in der ersten Lipidmembran enthalten ist, 20 bis 80% (Molverhältnis) der Gesamtmenge an Lipiden, die in der ersten Lipidmembran enthalten ist, ist.

6. Vektor gemäß einem der Ansprüche 1 bis 5, wobei die Menge des anionischen Lipids, die in der zweiten Lipidmembran enthalten ist, 10 bis 90% (Molverhältnis) der Gesamtmenge an Lipiden, die in der zweiten Lipidmembran enthalten ist, ist.

7. Vektor gemäß Anspruch 6, wobei die zweite Lipidmembran außerdem Dioleoylphosphatidylethanolamin enthält.

8. Vektor gemäß Anspruch 7, wobei die Menge an Dioleoylphosphatidylethanolamin, die in der zweiten Lipidmembran enthalten ist, 10 bis 90% (Molverhältnis) der Gesamtmenge an Lipiden, die in der zweiten Lipidmembran enthalten ist, ist.

**9.** Vektor gemäß einem der Ansprüche 1 bis 8, wobei das Membran-permeable Peptid ein Peptid ist, das eine Proteintransduktionsdomäne hat.

**10.** Vektor gemäß Anspruch 9, wobei die Proteintransduktionsdomäne Polyarginin ist.

**11.** Vektor gemäß Anspruch 10, wobei das Polyarginin aus 4 bis 20 fortlaufenden Argininresten besteht.

**12.** Vektor gemäß einem der Ansprüche 9 bis 11, wobei das Membran-permeable Peptid an der Oberfläche eines der ersten Lipidmembran und der zweiten Lipidmembran vorhanden ist.

**Revendications**

**1.** Vecteur destiné à la délivrance d'une substance cible dans un noyau, comprenant une structure membranaire lipidique ayant une première membrane lipidique contenant un lipide anionique et une deuxième membrane lipidique contenant un lipide anionique prévu en dehors de la première membrane lipidique, où le lipide anionique de la première membrane est choisi dans un groupe constitué par l'hémisuccinate de cholestéryle, l'acide phosphatidique et la cardiolipine et le lipide anionique de la deuxième membrane est choisi dans le groupe constitué par l'acide phosphatique, la cardiolipine, le dialkylphosphate et le diacylphosphate, et la première membrane lipidique et la deuxième membrane lipidique contiennent un peptide perméable à la membrane.

**2.** Vecteur selon la revendication 1, dans lequel la quantité de lipide anionique présent dans la première membrane lipidique est de 20 à 80 % (rapport en moles) de la quantité totale de lipides présents dans la première membrane lipidique.

**3.** Vecteur selon les revendications 1 ou 2, dans lequel le lipide anionique présent dans la première membrane lipidique est l'acide phosphatidique ou la cardiolipine et dans lequel la quantité de lipide anionique présent dans la première membrane lipidique est de 40 à 60 % (rapport en moles) de la quantité totale de lipides présents dans la première membrane lipidique.

**4.** Vecteur selon l'une quelconque des revendications 1 à 3, dans lequel la première membrane lipidique comprend en outre de la dioléoylphosphatidyl éthanolamine.

**5.** Vecteur selon la revendication 4, dans lequel la quantité de dioléoylphosphatidyl éthanolamine présente dans la première membrane lipidique est de 20 à 80 % (rapport en moles) de la quantité totale de lipides présents dans la première membrane lipidique.

**6.** Vecteur selon l'une quelconque des revendications 1 à 5, dans lequel la quantité de lipide anionique présent dans la deuxième membrane lipidique est de 10 à 90 % (rapport en moles) de la quantité totale de lipides présents dans la deuxième membrane lipidique.

**7.** Vecteur selon la revendication 6, dans lequel la deuxième membrane lipidique comprend en outre de la dioléoylphosphatidyl éthanolamine.

**8.** Vecteur selon la revendication 7, dans lequel la quantité de dioléoylphosphatidyl éthanolamine présente dans la deuxième membrane lipidique est de 10 à 90 % (rapport en moles) de la quantité totale de lipides présents dans la deuxième membrane lipidique.

**9.** Vecteur selon l'une quelconque des revendications 1 à 8, dans lequel le peptide membrane-perméable est un peptide ayant un domaine de transduction de la protéine.

**10.** Vecteur selon la revendication 9, dans lequel le domaine de transduction de la protéine est la polyarginine.

**11.** Vecteur selon la revendication 10, dans lequel la polyarginine est constituée de 4 à 20 résidus d'arginine consécutifs.

12. Vecteur selon l'une quelconque des revendications 9 à 11, dans lequel le peptide membrane-perméable est présent sur la surface de l'une quelconque de la première membrane lipidique et de la deuxième membrane lipidique.

Figure 1

Lipid : Chol = 2:1

binding rate (%)

Lipid : DOPE = 2:3

binding rate (%)

Figure 2

Figure 3

## Lipid : Chol = 2:1

## Lipid : DOPE = 2:3

Figure 4

## Lipid : Chol = 9:2

binding rate (%)

STR-R8(—)
STR-R8(+)

## Lipid : DOPE = 2:9

binding rate (%)

STR-R8(—)
STR-R8(+)

**Figure 5**

Lipid : Chol = 9:2

TF(%)

Lipid : DOPE = 2:9

TF(%)

Figure 6

Correlation between the ability to bind to nuclear membranes and
the ability to fuse with nuclear membranes

Figure 7

**1. EPC/Chol/STR-R8**

**2. DOPE/CHEMS/STR-R8**

**3. DOPE/PS/STR-R8**

**4. DOPE/PA/STR-R8**

Figure 8

DOPE/X=9:2

DOPE/X=5:5

Figure 9

EPC/X=5:5

Figure 10

**EPC/Chol/STR-R8**

**EPC/CHEMS**

**DOPE/PA**

**DOPE/CL**

Figure 11

Figure 12

Figure 13

Figure 14

Figure 15

Figure 16

EP 1 930 436 B1

Figure 17

（a）

（b）

（c）

（d）

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 06810956 A
- JP 2005289411 A

**Non-patent literature cited in the description**

- **Kogure et al.** *Journal of Controlled Release,* 2004, vol. 98, 317-323
- **Khalil Ikramy et al.** *YAKUGAKU ZASSHI,* 2004, vol. 124 (4), 113-116
- **Kogure K. et al.** Development of a non-viral multifunctional envelope-type nano device by a novel liquid film hydration method. *J. Control. Release,* 2004, vol. 98, 317-323
- **Patil S. D. et al.** Anionic liposomal delivery system for DNA transfection. *The AAPS Journal,* 2004, vol. 6 (4), e29
- **Moriguchi R. et al.** A multifunctional envelope-type nano device for novel gene delivery of siRNA plasmids. *International Journal of Pharmaceutics,* 2005, vol. 301 (1-2), 277-285
- **N. Oku.** Preparation and Experimentation of Liposome. Hirokawa-Shoten Ltd, 27-33
- *Biochem. Biophys. Acta.,* 1987, vol. 903, 123-131
- **S. Futaki.** Membrane permeable basic peptide. *PROTEIN, NUCLEIC ACID, AND ENZYME,* 2002, vol. 47, 1415-1419